(19) Europäisches Patentamt
European Patent Office
Office européen des brevets

(11) EP 0 581 272 B1

(12) **EUROPEAN PATENT SPECIFICATION**

(45) Date of publication and mention of the grant of the patent:
**08.05.1996 Bulletin 1996/19**

(51) Int. Cl.$^6$: **C07C 25/24**, C09K 19/18, C07C 255/50

(21) Application number: **93112087.7**

(22) Date of filing: **28.07.1993**

# (54) Tolane derivatives, liquid crystal compositions containing the derivatives and liquid crystal display device using the compositions

Tolan-Derivate, sie enthaltende flüssigkristalline Zusammensetzungen und flüssigkristalline Display-Vorrichtungen welche die Zusammensetzungen gebrauchen

Dérivés du tolane, compositions de cristaux liquides contenant ces dérivés et dispositifs d'affichage à cristaux liquides utilisant ces compositions

(84) Designated Contracting States:
**DE FR GB**

(30) Priority: **28.07.1992 JP 201333/92**
**18.09.1992 JP 250006/92**
**08.01.1993 JP 1933/93**
**10.03.1993 JP 49571/93**

(43) Date of publication of application:
**02.02.1994 Bulletin 1994/05**

(73) Proprietor: **SEIKO EPSON CORPORATION**
**Shinjuku-ku Tokyo-to (JP)**

(72) Inventors:
- **Yamada, Shuhei**
  **Suwa-shi, Nagano-ken (JP)**
- **Ikukawa, Shuji**
  **Suwa-shi, Nagano-ken (JP)**
- **Ito, Jun**
  **Suwa-shi, Nagano-ken (JP)**
- **Nakayama, Jitsuko**
  **Suwa-shi, Nagano-ken (JP)**

(74) Representative: **Hoffmann, Eckart, Dipl.-Ing.**
**Patentanwalt,**
**Bahnhofstrasse 103**
**82166 Gräfelfing (DE)**

(56) References cited:
**EP-A- 0 345 013** **DE-A- 4 005 882**

- **CHEMICAL ABSTRACTS, vol. 95, 21 December 1981, Columbus, Ohio, US; abstract no. 219871, page 494.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 581 272 B1

Printed by Rank Xerox (UK) Business Services
2.11.5/3.4

## Description

The present invention relates to a novel tolane derivative useful as a component of electro-optic display materials, a liquid crystal composition containing the derivative and a liquid crystal display device using the composition.

Liquid crystal display devices utilize the electro-optical effect of liquid crystals. The display modes currently used include the twisted nematic (hereinafter referred to as TN) mode and the super twisted nematic (hereinafter referred to as STN) mode the latter having a much larger twist angle than the former. The characteristics of the liquid crystals required for these display modes are as follows:

1. Colorless and thermal-, photo-, electrical- and chemical- stability;
2. Wide practical temperature range;
3. High-speed electro-optical response;
4. Low driving voltage;
5. Steep voltage-light transmittance characteristic, and small temperature dependency of the threshold voltage (hereinafter referred to as Vth); and
6. Wide visual angle.

A number of liquid crystal compounds which satisfy the characteristic 1 is well known. However, no single liquid crystal compound is known which satisfies all of the characteristics 2 to 6. Therefore, in order to satisfy these characteristics, liquid crystal compositions in which several kinds of nematic liquid crystal compounds or non-liquid crystal compounds are blended have been used.

For example, in order to satisfy the characteristic 2, a liquid crystal compound which has not only a low crystal phase-nematic liquid crystal phase transition point (hereinafter referred to as C-N point) but also a high nematic liquid crystal phase-isotropic liquid transition point (hereinafter referred to as N-I point) and consequently has a wide temperature range of the nematic liquid crystal phase, is required.

The response speed $\tau$, the viscosity $\eta$ and the cell gap d (the thickness of the liquid crystal layer) bear the following relationship:

$$\tau \propto \eta d^2$$

Thus, in order to satisfy the characteristic 3, i.e., to make the response speed fast, the cell gap d should be decreased.

In cells practically used, in order to prevent the occurrence of interference fringes on the surface of the cell which deteriorates the cell appearance, the product $\Delta n \cdot d$ ($\Delta n$ means the anisotropy of the refractive index) should have a certain value. Therefore, by using a material having a high $\Delta n$ value, the cell gap d can be made small and, thereby, the response speed increased.

In order to satisfy the characteristics 2 and 3 simultaneously, tolane derivatives (shown in Table 1 below) can be employed which are known as liquid crystal materials having not only a wide temperature range of the nematic liquid crystal phase but also a large $\Delta n$ value (described in e.g. Adv. in Liquid Crystal Research and Application (edited by L. Bata), Oxford: Pergamon Press; Budapest: Akademiai Kiado 1980, S. 1029; JP-A-305040/1989).

Table 1

| No. | Structural Formula | Transition Point | Literature |
|---|---|---|---|
| (i) | 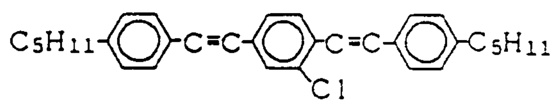 | C-N point: 100°C N-I point: 194°C | Adv. in Liquid Crystal Research and Application |
| (ii) | 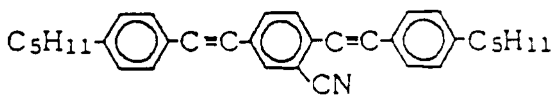 | C-N point: 77°C N-I point: 152°C | Adv. in Liquid Crystal Research and Application |
| (iii) | 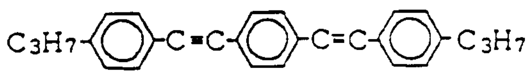 | C-N point: 179°C N-I point: 246°C | JP-A-305040/1989 |
| (iv) | 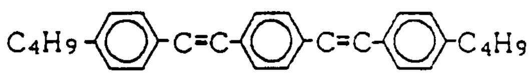 | C-N point: 152°C N-I point: 216°C | JP-A-305040/1989 |
| (v) | 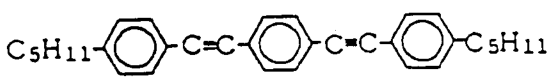 | C-N point: 151°C N-I point: 212°C | JP-A-305040/1989 |

It is described in the above literature that the compounds (i) and (ii) exhibit the nematic state in a wide temperature range and have no smectic state, and that the compounds (iii), (iv) and (v) have well-balanced desirable properties as liquid crystal components such as an extremely large Δn value, a low viscosity for compounds of three-ring structure and a high N-I point, respectively.

However, in the compounds (i) and (ii), the Δn is expected to be smaller, since they have chlorine atom or nitrile group in the side chains in their skeletons. On the other hand, the compounds (iii), (iv) and (v) have C-N points of 150°C or higher, and consequently it has to be assumed that these compounds lack sufficient compatibility with other liquid crystal compounds.

It is an object of the present invention to provide a novel liquid crystal compound which exhibits an excellent compatibility with several kinds of nematic liquid crystal compounds or non-liquid crystal compounds, and from which a novel liquid crystal composition having a wide practical temperature range and a large Δn value can be obtained by blending it with other compounds.

Another object of the present invention is to provide a liquid crystal display device having a wide practical temperature range and exhibiting a high-speed response by using said novel composition.

These objects are achieved with a tolane derivative, a liquid crystal composition and a liquid crystal display device, respectively, as claimed.

Specific embodiments of the invention will be described in detail below with reference Examples and to the single drawing which illustrates a liquid crystal display cell produced according to the present invention.

Compound (I) of the present invention can be prepared according to the following reaction steps:

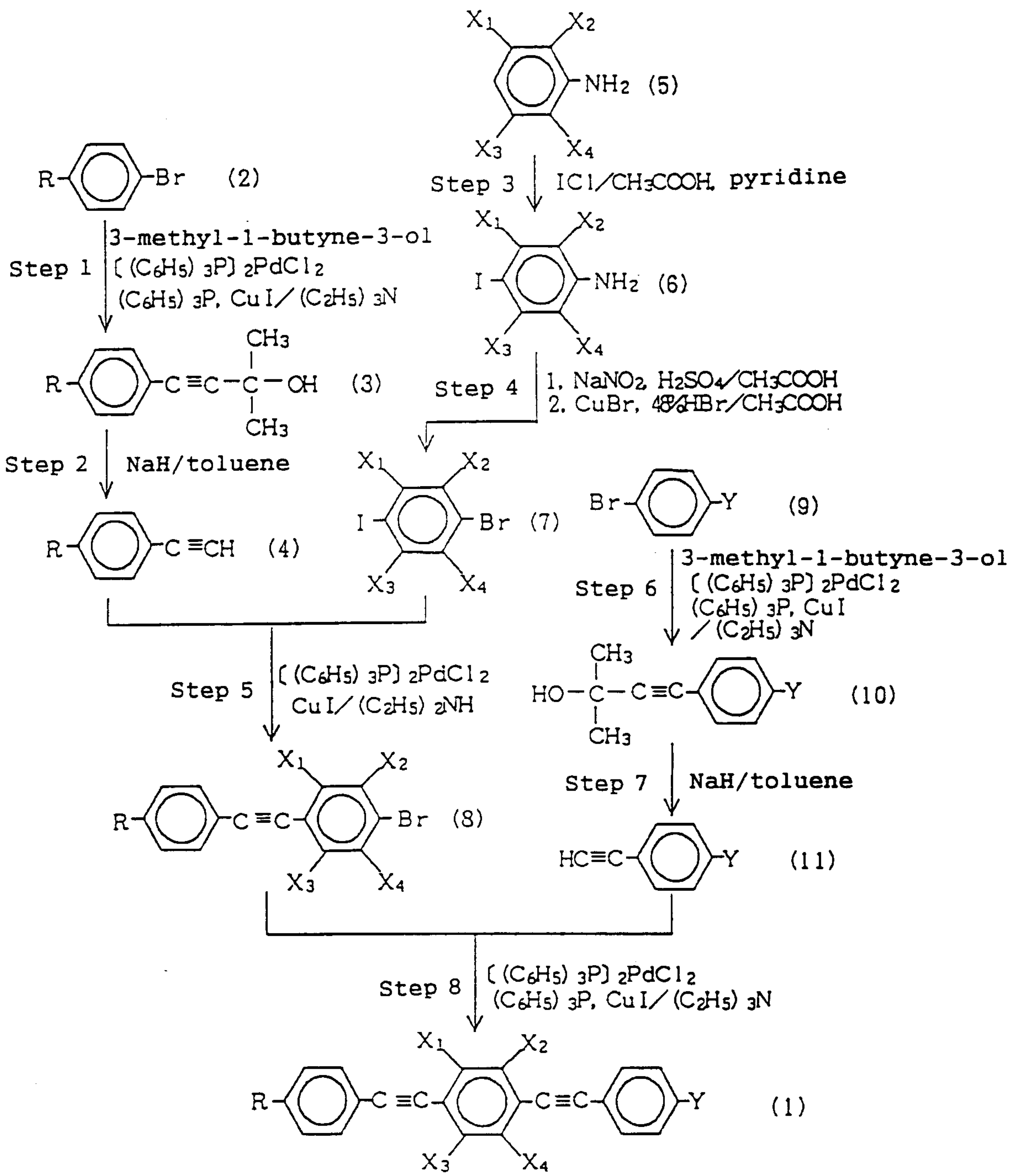


(In the formulae, R represents a straight-chain alkyl group having 1 to 10 carbon atoms, each of $X_1$, $X_2$, $X_3$ and $X_4$ represents a fluorine atom or a hydrogen atom wherein at least one $X_1$ to $X_4$ represents a fluorine atom, and Y represents a nitrile group or a straight-chain alkyl group having 1 to 10 carbon atoms.)

Step 1: Compound (2) is reacted with 3-methyl-1-butyne-3-ol in triethylamine in the presence of bis(triphenylphosphine)palladium(II) chloride, triphenylphosphine and copper(I) iodide to give Compound (3).

Step 2: Compound (3) is reacted with sodium hydride in toluene to give Compound (4).

Step 3: Compound (5) is reacted with iodine monochloride in acetic acid in the presence of pyridine to give Compound (6).

Step 4: Compound (6) is reacted with sodium nitrite and sulfuric acid in acetic acid to be converted into a diazonium salt, followed by reaction with copper (I) bromide and hydrobromic acid, to give Compound (7). In case where Compound (6) is commercially available, however, Step 3 is needless to carry out, and in case where Compound (7) is commercially available, Steps 3 and 4 are needless to carry out.

Step 5: Compound (4) is reacted with Compound (7) in diethylamine in the presence of bis(triphenylphosphine)palladium(II) chloride and copper(I) iodide to give Compound (8).

Step 6: Compound (9) is reacted with 3-methyl-1-butyne-3-ol in triethylamine in the presence of bis(triphenylphosphine)palladium(II) chloride, triphenylphosphine and copper(I) iodide to give Compound (10).

Step 7: Compound (10) is reacted with sodium hydride in toluene to give Compound (11).

Step 8: Compound (8) is reacted with Compound (11) in triethylamine in the presence of bis(triphenylphosphine)palladium(II) chloride, triphenylphosphine and copper(I) iodide to give Compound (1).

Examples of base components of the liquid crystal composition to be mixed with the tolane derivatives of the present invention are shown below, but are not limited thereto. The tolane derivatives of the present invention are characteristic in that they exhibit an excellent compatibility with all of the conventional liquid crystal compounds or their analogues and the resulting liquid crystal compositions have wide practical temperature ranges and large Δn values.

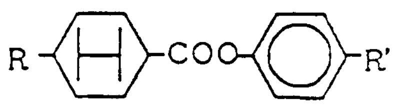
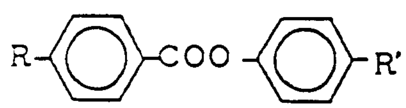

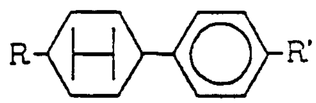
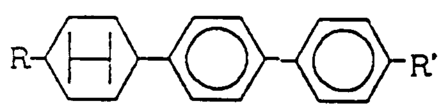

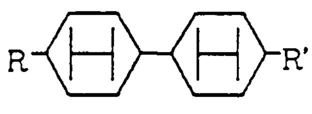
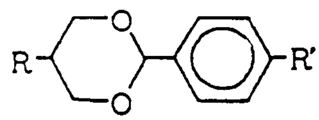

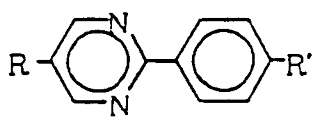
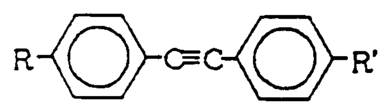

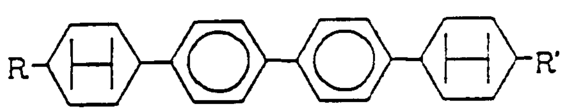
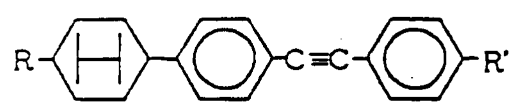

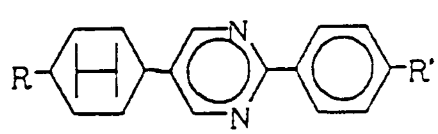
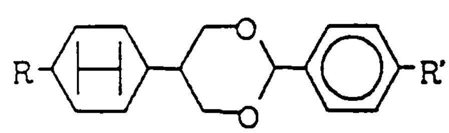

(In the formulae, each of R and R' represents an alkyl group, an alkoxy group, an alkoxymethylene group, a nitrile group or a flourine atom; phenylene groups may have a halogen substituent at 2-position or 3-position; and the cyclohexane rings are in trans configuration.)

The blending ratio of the compounds of the present invention in a liquid crystal composition is in the range of 1 to 50 wt%. It is particularly preferable to be in the range of 3 to 30 wt% in consideration of precipitation of crystals in the low temperature region.

The liquid crystal display apparatus which uses the liquid crystal composition containing at least one kind of the compounds of the present invention is highly suitable for a liquid crystal display apparatus which utilizes time-sharing addressing mode. In particular, TN mode- and STN mode-liquid crystal display devices prepared by using the said compositions can be driven by high time-sharing addressing.

## EXAMPLES

To further illustrate the present invention, the following Examples will be given.

Example 1: [Synthesis of Compound (1-a)]

Preparation of 1-(4'-propylphenylethynyl)-4-(4"-pentylphenylethynyl)-2-fluorobenzene

Step 1: 1-Bromo-4-propylbenzene (60 g), 3-methyl-1-butyne-3-ol (38 g), triphenylphosphine (1.3 g) and bis(triphenylphosphine)palladium(II) chloride (0.7 g) were dissolved in triethylamine (260 ml) under nitrogen atmosphere, and then copper(I) iodide (0.2 g) was added thereto. The mixture was stirred at room temperature for 1 hour, and then further stirred at 90°C for 5 hours. The resulting precipitated crystals were filtered off, and triethylamine was then distilled off, followed by extraction with chloroform. The resultant was washed with 10 % hydrochloric acid twice, and further with water twice, followed by distilling off chloroform therefrom. The residue was purified by the use of silica gel-chloroform column chromatography to give 3-methyl-1-(4'-propylphenyl)-1-butyne-3-ol (37 g).

Step 2: 3-Methyl-1-(4'-propylphenyl)-1-butyne-3-ol (33 g) was dissolved in toluene (320 ml) under nitrogen atmosphere, and then sodium hydride (60 % in paraffine liquid; 2 g) was added thereto. The mixture was stirred at 60°C for 6 hours. The reaction solution was poured into water (300 ml), and then extracted with chloroform, followed by washing with water 3 times. After distilling off toluene and chloroform, the resultant was distilled under reduced pressure (b.p. 60 to 63°C/4 mmHg) to give 4-propylphenylacetylene (16 g).

Steps 3 and 4: These steps were needless to carry out, since 4-bromo-2-fluoro-1-iodobenzene is commercially available.

Step 5: 4-Bromo-2-fluoro-1-iodobenzene (33 g) was dissolved in diethylamine (37 ml) under nitrogen atmosphere, and then bis(triphenylphosphine)palladium(II) chloride (0.1 g) and copper(I) iodide (0.1 g) were added thereto, followed by stirring. The flask was cooled to 5°C or below, and then 4-propylphenylacetylene (16 g) was added dropwise thereto. After stirring at room temperature for 5 hours, the reaction solution was poured into a mixture of concentrated hydrochloric acid (23 ml) and ice (150 g). After extraction with chloroform, the resultant was washed with water twice, followed by distilling off chloroform therefrom. The residue was distilled under reduced pressure (b.p. 190 to 200°C/4 mmHg), and then recrystallized from a mixed solvent of acetone and methanol to give 4-bromo-2-fluoro-4'-propyltolane (23 g).

Step 6: 1-Bromo-4-pentylbenzene (102 g), 3-methyl-1-butyne-3-ol (38 g), triphenylphosphine (2 g) and bis(triphenylphosphine)palladium(II) chloride (1 g) were dissolved in triethylamine (390 ml) under nitrogen atmosphere, and then copper(I) iodide (0.3 g) was added thereto. After stirring at room temperature for 1 hour, the reaction mixture was further stirred at 90°C for 5 hours. The resulting precipitated crystals were filtered off, and thentriethylamine was distilled off therefrom, followed by extracting with chloroform. The resultant was washed with 10 % hydrochloric acid twice, and further with water twice, and then chloroform was distilled off therefrom. The residue was purified by use of silica gel-chloroform column chromatography to give 3-methyl-1-(4'-pentylphenyl)-1-butyne-3-ol (82 g).

Step 7: 3-Methyl-1-(4'-pentylphenyl)-1-butyne-3-ol (82 g) was dissolved in toluene (900 ml) under nitrogen atmosphere, and then sodium hydride (60 % in paraffin liquid; 5.6 g) was added thereto, followed by stirring at 60°C for 6 hours. The reaction solution was poured into water (500 ml), and extracted with chloroform, followed by washing with water 3 times. After distilling off toluene and chloroform, the resultant was distilled under reduced pressure (b.p. 85 to 90°C/3 mmHg) to give 4-pentylphenylacetylene (48 g).

Step 8: 4-Bromo-2-fluoro-4'-propyltolane (3.8 g), 4-pentylphenylacetylene (2.1 g), triphenylphosphine (0.05 g) and bis(triphenylphosphine)palladium(II) chloride (0.02 g) were dissolved in triethylamine (27 ml) under nitrogen atmosphere, and then copper(I) iodide (0.01 g) was added thereto, followed by reflux for 5 hours. The reaction solution was poured into water (300 ml), and then extracted with chloroform. After washing the resultant with water twice, chloroform was distilled off therefrom. The residue was purified by use of silica gel-chloroform column chromatography, and then recrystallized from acetone to give 1-(4'-propylphenylethynyl)-4-(4"-pentylphenylethynyl)-2-fluorobenzene (1.4 g). The C-N point and the N-I-point of the compound were 81.3°C and 202.7°C, respectively.

The following compounds were synthesized in the same manner as the above method:
1,4-bis(4'-methylphenylethynyl)-2-fluorobenzene
1-(4'-methylphenylethynyl)-4-(4"-ethylphenylethynyl)-2-fluorobenzene
1-(4'-methylphenylethynyl)-4-(4"-propylphenylethynyl)-2-fluorobenzene
1-(4'-methylphenylethynyl)-4-(4"-butylphenylethynyl)-2-fluorobenzene
1-(4'-methylphenylethynyl)-4-(4"-pentylphenylethynyl)-2-fluorobenzene
1-(4'-methylphenylethynyl)-4-(4"-hexylphenylethynyl)-2-fluorobenzene
1-(4'-methylphenylethynyl)-4-(4"-heptylphenylethynyl)-2-fluorobenzene
1-(4'-methylphenylethynyl)-4-(4"-octylphenylethynyl)-2-fluorobenzene
1-(4'-methylphenylethynyl)-4-(4"-nonylphenylethynyl)-2-fluorobenzene
1-(4'-methylphenylethynyl)-4-(4"-decylphenylethynyl)-2-fluorobenzene

1-(4'-ethylphenylethynyl)-4-(4''-methylphenylethynyl)-2-fluorobenzene
1,4-bis(4'-ethylphenylethynyl)-2-fluorobenzene
1-(4'-ethylphenylethynyl)-4-(4''-propylphenylethynyl)-2-fluorobenzene
1-(4'-ethylphenylethynyl)-4-(4''-butylphenylethynyl)-2-fluorobenzene
1-(4'-ethylphenylethynyl)-4-(4''-pentylphenylethynyl)-2-fluorobenzene
1-(4'-ethylphenylethynyl)-4-(4''-hexylphenylethynyl)-2-fluorobenzene
1-(4'-ethylphenylethynyl)-4-(4''-heptylphenylethynyl)-2-fluorobenzene
1-(4'-ethylphenylethynyl)-4-(4''-octylphenylethynyl)-2-fluorobenzene
1-(4'-ethylphenylethynyl)-4-(4''-nonylphenylethynyl)-2-fluorobenzene
1-(4'-ethylphenylethynyl)-4-(4''-decylphenylethynyl)-2-fluorobenzene
1-(4'-propylphenylethynyl)-4-(4''-methylphenylethynyl)-2-fluorobenzene
1-(4'-propylphenylethynyl)-4-(4''-ethylphenylethynyl)-2-fluorobenzene
C-N point 150.3°C, N-I point 214.1°C
1,4-bis(4'-propylphenylethynyl)-2-fluorobenzene
C-N point 147.0°C, N-I point 220.0°C
1-(4'-propylphenylethynyl)-4-(4''-butylphenylethynyl)-2-fluorobenzene
C-N point 112.4°C, N-I point 203.7°C
1-(4'-propylphenylethynyl)-4-(4''-hexylphenylethynyl)-2-fluorobenzene
1-(4'-propylphenylethynyl)-4-(4''-heptylphenylethynyl)-2-fluorobenzene
1-(4'-propylphenylethynyl)-4-(4''-octylphenylethynyl)-2-fluorobenzene
1-(4'-propylphenylethynyl)-4-(4''-nonylphenylethynyl)-2-fluorobenzene
1-(4'-propylphenylethynyl)-4-(4''-decylphenylethynyl)-2-fluorobenzene
1-(4'-butylphenylethynyl)-4-(4''-methylphenylethynyl)-2-fluorobenzene
1-(4'-butylphenylethynyl)-4-(4''-ethylphenylethynyl)-2-fluorobenzene
1-(4'-butylphenylethynyl)-4-(4''-propylphenylethynyl)-2-fluorobenzene
C-N point 112.6°C, N-I point 201.2°C
1,4-bis(4'-butylphenylethynyl)-2-fluorobenzene
C-N point 91.5°C, N-I point 185.6°C
1-(4'-butylphenylethynyl)-4-(4''-pentylphenylethynyl)-2-fluorobenzene
C-N point 78.5°C, N-I point 186.9°C
1-(4'-butylphenylethynyl)-4-(4''-hexylphenylethynyl)-2-fluorobenzene
1-(4'-butylphenylethynyl)-4-(4''-heptylphenylethynyl)-2-fluorobenzene
1-(4'-butylphenylethynyl)-4-(4''-octylphenylethynyl)-2-fluorobenzene
1-(4'-butylphenylethynyl)-4-(4''-nonylphenylethynyl)-2-fluorobenzene
1-(4'-butylphenylethynyl)-4-(4''-decylphenylethynyl)-2-fluorobenzene
1-(4'-pentylphenylethynyl)-4-(4''-methylphenylethynyl)-2-fluorobenzene
1-(4'-pentylphenylethynyl)-4-(4''-ethylphenylethynyl)-2-fluorobenzene
1-(4'-pentylphenylethynyl)-4-(4''-propylphenylethynyl)-2-fluorobenzene
C-N point 88.3°C, N-I point 203.0°C
1-(4'-pentylphenylethynyl)-4-(4''-butylphenylethynyl)-2-fluorobenzene
C-N point 82.4°C, N-I point 187.7°C
1,4-bis(4'-pentylphenylethynyl)-2-fluorobenzene
C-N point 89.2°C, N-I point 189.0°C
1-(4'-pentylphenylethynyl)-4-(4''-hexylphenylethynyl)-2-fluorobenzene
1-(4'-pentylphenylethynyl)-4-(4''-heptylphenylethynyl)-2-fluorobenzene
1-(4'-pentylphenylethynyl)-4-(4''-octylphenylethynyl)-2-fluorobenzene
1-(4'-pentylphenylethynyl)-4-(4''-nonylphenylethynyl)-2-fluorobenzene
1-(4'-pentylphenylethynyl)-4-(4''-decylphenylethynyl)-2-fluorobenzene
1-(4'-hexylphenylethynyl)-4-(4''-methylphenylethynyl)-2-fluorobenzene
1-(4'-hexylphenylethynyl)-4-(4''-ethylphenylethynyl)-2-fluorobenzene
1-(4'-hexylphenylethynyl)-4-(4''-propylphenylethynyl)-2-fluorobenzene
1-(4'-hexylphenylethynyl)-4-(4''-butylphenylethynyl)-2-fluorobenzene
1-(4'-hexylphenylethynyl)-4-(4''-pentylphenylethynyl)-2-fluorobenzene
1,4-bis (4'-hexylphenylethynyl)-2-fluorobenzene
1-(4'-hexylphenylethynyl)-4-(4''-heptylphenylethynyl)-2-fluorobenzene
1-(4'-hexylphenylethynyl)-4-(4''-octylphenylethynyl)-2-fluorobenzene
1-(4'-hexylphenylethynyl)-4-(4''-nonylphenylethynyl)-2-fluorobenzene
1-(4'-hexylphenylethynyl)-4-(4''-decylphenylethynyl)-2-fluorobenzene
1-(4'-heptylphenylethynyl)-4-(4''-methylphenylethynyl)-2-fluorobenzene

1-(4'-heptylphenylethynyl)-4-(4"-ethylphenylethynyl)-2-fluorobenzene
1-(4'-heptylphenylethynyl)-4-(4"-propylphenylethynyl)-2-fluorobenzene
1-(4'-heptylphenylethynyl)-4-(4"-butylphenylethynyl)-2-fluorobenzene
1-(4'-heptylphenylethynyl)-4-(4"-pentylphenylethynyl)-2-fluorobenzene
1-(4'-heptylphenylethynyl)-4-(4"-hexylphenylethynyl)-2-fluorobenzene
1,4-bis(4'-heptylphenylethynyl)-2-fluorobenzene
1-(4'-heptylphenylethynyl)-4-(4"-octylphenylethynyl)-2-fluorobenzene
1-(4'-heptylphenylethynyl)-4-(4"-nonylphenylethynyl)-2-fluorobenzene
1-(4'-heptylphenylethynyl)-4-(4"-decylphenylethynyl)-2-fluorobenzene
1-(4'-octylphenylethynyl)-4-(4"-methylphenylethynyl)-2-fluorobenzene
1-(4'-octylphenylethynyl)-4-(4"-ethylphenylethynyl)-2-fluorobenzene
1-(4'-octylphenylethynyl)-4-(4"-propylphenylethynyl)-2-fluorobenzene
1-(4'-octylphenylethynyl)-4-(4"-butylphenylethynyl)-2-fluorobenzene
1-(4'-octylphenylethynyl)-4-(4"-pentylphenylethynyl)-2-fluorobenzene
1-(4'-octylphenylethynyl)-4-(4"-hexylphenylethynyl)-2-fluorobenzene
1-(4'-octylphenylethynyl)-4-(4"-heptylphenylethynyl)-2-fluorobenzene
1,4-bis(4'-octylphenylethynyl)-2-fluorobenzene
1-(4'-octylphenylethynyl)-4-(4"-nonylphenylethynyl)-2-fluorobenzene
1-(4'-octylphenylethynyl)-4-(4"-decylphenylethynyl)-2-fluorobenzene
1-(4'-nonylphenylethynyl)-4-(4"-methylphenylethynyl)-2-fluorobenzene
1-(4'-nonylphenylethynyl)-4-(4"-ethylphenylethynyl)-2-fluorobenzene
1-(4'-nonylphenylethynyl)-4-(4"-propylphenylethynyl)-2-fluorobenzene
1-(4'-nonylphenylethynyl)-4-(4"-butylphenylethynyl)-2-fluorobenzene
1-(4'-nonylphenylethynyl)-4-(4"-pentylphenylethynyl)-2-fluorobenzene
1-(4'-nonylphenylethynyl)-4-(4"-hexylphenylethynyl)-2-fluorobenzene
1-(4'-nonylphenylethynyl)-4-(4"-heptylphenylethynyl)-2-fluorobenzene
1-(4'-nonylphenylethynyl)-4-(4"-octylphenylethynyl)-2-fluorobenzene
1,4-bis (4'-nonylphenylethynyl)-2-fluorobenzene
1-(4'-nonylphenylethynyl)-4-(4"-decylphenylethynyl)-2-fluorobenzene
1-(4'-decylphenylethynyl)-4-(4"-methylphenylethynyl)-2-fluorobenzene
1-(4'-decylhenylethynyl)-4-(4"-ethylphenylethynyl)-2-fluorobenzene
1-(4'-decylphenylethynyl)-4-(4"-propylphenylethynyl)-2-fluorobenzene
1-(4'-decylphenylethynyl)-4-(4"-butylphenylethynyl)-2-fluorobenzene
1-(4'-decylphenylethynyl)-4-(4"-pentylphenylethynyl)-2-fluorobenzene
1-(4'-decylphenylethynyl)-4-(4"-hexylphenylethynyl)-2-fluorobenzene
1-(4'-decylphenylethynyl)-4-(4"-heptylphenylethynyl)-2-fluorobenzene
1-(4'-decylphenylethynyl)-4-(4"-octylphenylethynyl)-2-fluorobenzene
1,4-bis(4'-decylphenylethynyl)-2-fluorobenzene
1-(4'-decylphenylethynyl)-4-(4"-nonylphenylethynyl)-2-fluorobenzene

Example 2: [Synthesis of Compound (1-b)]

Preparation of 1-(4'-pentylphenylethynyl)-4-(4"-cyanophenylethynyl)-2-fluorobenzene

Steps 1 and 2: 4-Pentylphenylacetylene was synthesized in the same manner as Steps 6 and 7 in Example 1.

Steps 3 and 4: These steps were needless to carry out, since 4-bromo-2-fluoro-1-iodobenzene is commercially available.

Step 5: 4-Bromo-2-fluoro-1-iodobenzene (57 g) was dissolved in diethylamine (66 ml) under nitrogen atmosphere, and then bis(triphenylphosphine)palladium(II) chloride (0.5 g) and copper(I) iodide (0.5 g) were added thereto, followed by stirring. The flask was cooled to 5°C or below, and then 4-pentylphenylacetylene (36 g) was added dropwise thereto. After stirring at room temperature for 5 hours, the reaction solution was poured into a mixture of concentrated hydrochloric acid (40 ml) and ice (30 g). After extraction with chloroform, the resultant was washed with water twice, followed by distilling off chloroform therefrom. The residue was distilled under reduced pressure (b.p. 205 to 210°C/3 mmHg), and then recrystallized from a mixed solvent of acetone and methanol to give 4-bromo-2-fluoro-4'-pentyltolane (27 g).

Step 6: 4-Bromobenzonitrile (30 g), 3-methyl-1-butyne-3-ol (13.4 g), triphenylphosphine (0.7 g) and bis(triphenylphosphine)palladium(II) chloride (0.3 g) were dissolved in triethylamine (140 ml) under nitrogen atmosphere, and then copper(I) iodide (0.1 g) was added thereto. After stirring at room temperature for 1 hour, the reaction mixture was further stirred at 90°C for 5 hours. The resulting precipitated crystals were filtered off, and then triethylamine was distilled off therefrom, followed by extracting with chloroform. The resultant was washed with 10 % hydrochloric acid twice, and

further with water twice, and then chloroform was distilled off therefrom. The residue was distilled under reduced pressure (140 to 160°C/5 mmHg) to give 3-methyl-1-(4'-cyanophenyl)-1-butyne-3-ol (23.5 g).

Step 7: 3-Methyl-1-(4'-cyanophenyl)-1-butyne-3-ol (23.5 g) was dissolved in toluene (250 ml) under nitrogen atmosphere, and then sodium hydride (1.6 g) was added thereto, followed by stirring at 80 to 90°C for 4 hours. The reaction solution was poured into water (300 ml), and then extracted with chloroform, followed by washing with water 3 times. After distilling off toluene and chloroform, the resultant was recrystallized from methanol to give 4-cyanophenylacetylene (9.9 g).

Step 8: 4-Bromo-2-fluoro-4'-pentyltolane (5.2 g), 4-cyanophenylacetylene (1.9 g), triphenylphosphine (0.06 g) and bis(triphenylphosphine)palladium(II) chloride (0.04 g) were dissolved in triethylamine (33 ml) under nitrogen atmosphere, and then copper(I) iodide (0.01 g) was added thereto, followed by reflux for 5 hours. The reaction solution was poured into water (300 ml), and then extracted with chloroform. After washing the resultant with water twice, chloroform was distilled off therefrom. The residue was purified by use of silica gel-chloroform column chromatography, and then recrystallized from a mixed solvent of acetone and chloroform to give 1-(4'-pentylphenylethynyl)-4-(4"-cyanophenylethynyl)-2-fluorobenzene (2.7 g). The C-N point and the N-I-point of the compound were 150.5°C and 256.5°C, respectively.

The following compounds were synthesized in the same manner as the above method:

1-(4'-methylphenylethynyl)-4-(4"-cyanophenylethynyl)-2-fluorobenzene
1-(4'-ethylphenylethynyl)-4-(4"-cyanophenylethynyl)-2-fluorobenzene
1-(4'-propylphenylethynyl)-4-(4"-cyanophenylethynyl)-2-fluorobenzene
1-(4'-butylphenylethynyl)-4-(4"-cyanophenylethynyl)-2-fluorobenzene
1-(4'-hexylphenylethynyl)-4-(4"-cyanophenylethynyl)-2-fluorobenzene
1-(4'-heptylphenylethynyl)-4-(4"-cyanophenylethynyl)-2-fluorobenzene
1-(4'-octylphenylethynyl)-4-(4"-cyanophenylethynyl)-2-fluorobenzene
1-(4'-nonylphenylethynyl)-4-(4"-cyanophenylethynyl)-2-fluorobenzene
1-(4'-decylphenylethynyl)-4-(4"-cyanophenylethynyl)-2-fluorobenzene

Example 3: [Synthesis of Compound (1-c)]

Preparation of 4-(4'-propylphenylethynyl)-1-(4"-cyanophenylethynyl)-2-fluorobenzene

Steps 1 and 2: 4-Pentylphenylacetylene was synthesized in the same manner as Steps 6 and 7 in Example 1.

Step 3: This step was needless to carry out, since 2-fluoro-4-iodoaniline is commercially available.

Step 4: Sodium nitrite (51.2 g) was dissolved in sulfuric acid (390 ml), and then acetic acid (454 ml) was added thereto at 10°C or below. The mixed solution was kept at 20 to 25°C, and 2-fluoro-4-iodoaniline (124 g) was added during 1 hour, followed by stirring for 2 hours. The reaction solution was added dropwise to a mixed solution of copper bromide (130 g) with 48 % hydrobromic acid (390 ml), and stirred overnight. Then, water (1000 ml) was added to the solution, and the resulting solution was extracted with chloroform, followed by washing with water 3 times. After distilling off chloroform, the resultant was distilled under reduced pressure (b.p. 120 to 125°C/13 mmHg), and then recrystallized from methanol to give 1-bromo-2-fluoro-4-iodobenzene (114 g).

Step 5: 1-Bromo-2-fluoro-4-iodobenzene (15 g) was dissolved in diethylamine (17 ml) under nitrogen atmosphere, and then bis(triphenylphosphine)palladium(II) chloride (0.12 g) and copper(I) iodide (0.12 g) were added thereto, followed by stirring. The flask was cooled to 5°C or below, and then 4-propylphenylacetylene (8 g) was added dropwise thereto. After stirring at room temperature for 5 hours, the reaction solution was poured into a mixture of concentrated hydrochloric acid (10 ml) and ice (50 g). After extraction with chloroform, the resultant was washed with water twice, followed by distilling off chloroform. The residue was recrystallized from a mixed solvent of acetone and methanol to give 4-bromo-3-fluoro-4'-propyltolane (11 g).

Steps 6 and 7: 4-Cyanophenylacetylene was synthesized in the same manner as Steps 6 and 7 in Example 2.

Step 8: 4-Bromo-3-fluoro-4'-propyltolane (4.8 g), 4-cyanophenylacetylene (1.9 g), triphenylphosphine (0.06 g) and bis(triphenylphosphine)palladium(II) chloride (0.04 g) were dissolved in triethylamine (33 ml) under nitrogen atmosphere, and then copper(I) iodide (0.01 g) was added thereto, followed by reflux for 5 hours. The reaction solution was poured into water (300 ml), and then extracted with chloroform. After washing the resultant with water twice, chloroform was distilled off therefrom. The residue was purified by use of silica gel-chloroform column chromatography, and then recrystallized from a mixed solvent of acetone and chloroform, to give 4-(4'-propylphenylethynyl)-1-(4"-cyanophenylethynyl)-2-fluorobenzene (3.4 g). The C-N point and the N-I-point of the compound were 163.6°C and 275.3°C, respectively.

The following compounds were synthesized in the same manner as the above method:

4-(4'-methylphenylethynyl)-1-(4"-cyanophenylethynyl)-2-fluorobenzene
4-(4'-ethylphenylethynyl)-1-(4"-cyanophenylethynyl)-2-fluorobenzene
4-(4'-butylphenylethynyl)-1-(4"-cyanophenylethynyl)-2-fluorobenzene
4-(4'-pentylphenylethynyl)-1-(4"-cyanophenylethynyl)-2-fluorobenzene
4-(4'-hexylphenylethynyl)-1-(4"-cyanophenylethynyl)-2-fluorobenzene

4-(4'-heptylphenylethynyl)-1-(4"-cyanophenylethynyl)-2-fluorobenzene
4-(4'-octylphenylethynyl)-1-(4"-cyanophenylethynyl)-2-fluorobenzene
4-(4'-nonylphenylethynyl)-1-(4"-cyanophenylethynyl)-2-fluorobenzene
4-(4'-decylphenylethynyl)-1-(4"-cyanophenylethynyl)-2-fluorobenzene

Example 4: [Synthesis of Compound (1-d)]

Preparation of 5-(4'-propylphenylethynyl)-2-(4"-butylphenylethynyl)-1,3-difluorobenzene

Steps 1 and 2: 4-Propylphenylacetylene was synthesized in the same manner as Steps 1 and 2 in Example 1.

Step 3: 2,6-Difluoroaniline (38 g) was dissolved in acetic acid (120 ml), and then pyridine (25 g) was added thereto, followed by stirring. Then, a mixture of iodine monochloride (50 g) with acetic acid (30 ml) was added dropwise thereto. After stirring at room temperature for 1 hour, the reaction solution was further stirred at 70 to 80°C for 2 hours. Then, the reaction solution was poured into water, and the precipitated crystals were filtered off, followed by washing with water. The resulting crystals were dissolved in chloroform, and then washed with water twice, further with 10 % potassium hydroxide aqueous solution twice, and furthermore with water twice, followed by distilling off chloroform. The residue was distilled under reduced pressure (b.p. 120 to 130°C/20 mmHg), and then recrystallized from methanol to give 4-iodo-2,6-difluoroaniline (44 g).

Step 4: Sodium nitrite (17 g) was dissolved in sulfuric acid (130 ml), and then acetic acid (150 ml) was added thereto at 10°C or below. The mixed solution was kept at 20 to 25°C, and 4-iodo-2,6-difluoroaniline (44 g) was added thereto during 1 hour, followed by stirring for 2 hours. The reaction solution was added dropwise to a mixed solution of copper (I) bromide (43 g) with 48 % hydrobromic acid (125 ml), and stirred overnight. Then, water (1000 ml) was added to the solution, and the resulting solution was extracted with chloroform, followed by washing with water 3 times. After distilling off chloroform, the resultant was recrystallized from methanol to give 2-bromo-1,3-difluoro-4-iodobenzene (38 g).

Step 5: 2-Bromo-1,3-difluoro-4-iodobenzene (20 g) was dissolved in diethylamine (30 ml) under nitrogen atmosphere, and then bis(triphenylphosphine)palladium(II) chloride (0.06 g) and copper(I) iodide (0.06 g) were added thereto, followed by stirring. The flask was cooled to 5°C or below, and then 4-propylphenylacetylene (10 g) was added dropwise thereto. After stirring at room temperature for 5 hours, the reaction solution was poured into a mixture of concentrated hydrochloric acid (15 ml) and ice (30 g). After extraction with chloroform, the resultant was washed with water twice, followed by distilling off chloroform. The residue was distilled under reduced pressure (b.p. 185 to 192°C/4 mmHg), and then recrystallized from a mixed solvent of acetone and methanol to give 4-bromo-3,5-difluoro-4'-propyltolane (12 g).

Step 6: 1-Bromo-4-butylbenzene (235 g), 3-methyl-1-butyne-3-ol (139 g), triphenylphosphine (4.8 g) and bis(triphenylphosphine)palladium(II) chloride (2.8 g) were dissolved in triethylamine (1,000 ml), and then copper(I) iodide (0.8 g) was added thereto. After stirring at room temperature for 1 hour, the reaction mixture was further stirred at 90°C for 5 hours. The resulting precipitated crystals were filtered off, and then triethylamine was distilled off therefrom, followed by extracting with chloroform. The resultant was washed with 10 % hydrochloric acid twice, and further with water twice, and then chloroform was distilled off therefrom. The residue was purified by use of silica gel-chloroform column chromatography to give 3-methyl-1-(4'-butylphenyl)-1-butyne-3-ol (100 g).

Step 7: 3-Methyl-1-(4'-butylphenyl)-1-butyne-3-ol (100 g) was dissolved in toluene (900 ml) under nitrogen atmosphere, and then sodium hydride (60 % in paraffin liquid; 5.7 g) was added thereto, followed by stirring at 60°C for 6 hours. The reaction solution was poured into water (500 ml), and then extracted with chloroform, followed by washing with water 3 times. After distilling off toluene and chloroform, the resultant was distilled under reduced pressure (b.p. 62 to 65°C/3 mmHg) to give 4-butylphenylacetylene (63 g).

Step 8: 4-Bromo-3,5-difluoro-4'-propyltolane (5.0 g), 4-butylphenylacetylene (2.4 g), triphenylphosphine (0.06 g) and bis(triphenylphosphine)palladium(II) chloride (0.04 g) were dissolved in triethylamine (33 ml) under nitrogen atmosphere, and then copper(I) iodide (0.01 g) was added thereto, followed by reflux for 5 hours. The reaction solution was poured into a mixture of concentrated hydrochloric acid (16 ml) and ice (30 g), and then extracted with chloroform. After washing the extract solution with water twice, chloroform was distilled off therefrom. The residue was purified by use of silica gel-chloroform column chromatography, and then recrystallized from acetone to give 5-(4'-propylphenylethynyl)-2-(4"-butylphenylethynyl)-1,3-difluorobenzene (3.5 g). The C-N point and the N-I point of the compound were 120.4°C and 181.9°C, respectively.

The following compounds were synthesized in the same manner as the above method:
5-(4'-methylphenylethynyl)-2-(4"-methylphenylethynyl)-1,3-difluorobenzene
5-(4'-methylphenylethynyl)-2-(4"-ethylphenylethynyl)-1,3-difluorobenzene
5-(4'-methylphenylethynyl)-2-(4"-propylphenylethynyl)-1,3-difluorobenzene
5-(4'-methylphenylethynyl)-2-(4"-butylphenylethynyl)-1,3-difluorobenzene
5-(4'-methylphenylethynyl)-2-(4"-pentylphenylethynyl)-1,3-difluorobenzene
5-(4'-methylphenylethynyl)-2-(4"-hexylphenylethynyl)-1,3-difluorobenzene
5-(4'-methylphenylethynyl)-2-(4"-heptylphenylethynyl)-1,3-difluorobenzene

5-(4'-methylphenylethynyl)-2-(4"-octylphenylethynyl)-1,3-difluorobenzene
5-(4'-methylphenylethynyl)-2-(4"-nonylphenylethynyl)-1,3-difluorobenzene
5-(4'-methylphenylethynyl)-2-(4"-decylphenylethynyl)-1,3-difluorobenzene
5-(4'-ethylphenylethynyl)-2-(4"-methylphenylathynyl)-1,3-difluorobenzene
5-(4'-ethylphenylethynyl)-2-(4"-ethylphenylethynyl)-1,3-difluorobenzene
5-(4'-ethylphenylethynyl)-2-(4"-propylphenylethynyl)-1,3-difluorobenzene
5-(4'-ethylphenylethynyl)-2-(4"-butylphenylethynyl)-1,3-difluorobenzene
5-(4'-ethylphenylethynyl)-2-(4"-pentylphenylethynyl)-1,3-difluorobenzene
5-(4'-ethylphenylethynyl)-2-(4"-hexylphenylethynyl)-1,3-difluorobenzene
5-(4'-ethylphenylethynyl)-2-(4"-heptylphenylathynyl)-1,3-difluorobenzene
5-(4'-ethylphenylethynyl)-2-(4"-octylphenylethynyl)-1,3-difluorobenzene
5-(4'-ethylphenylethynyl)-2-(4"-nonylphenylethynyl)-1,3-difluorobenzene
5-(4'-ethylphenylethynyl)-2-(4"-decylphenylethynyl)-1,3-difluorobenzene
5-(4'-propylphenylethynyl)-2-(4"-methylphenylethynyl)-1,3-difluorobenzene
5-(4'-propylphenylethynyl)-2-(4"-ethylphenylethynyl)-1,3-difluorobenzene
5-(4'-propylphenylethynyl)-2-(4"-propylphenylethynyl)-1,3-difluorobenzene
C-N point 155.9°C, N-I point 196.9°C
5-(4'-propylphenylethynyl)-2-(4"-pentylphenylethynyl)-1,3-difluorobenzene
C-N point 79.6°C, N-I point 182.7°C
5-(4'-propylphenylethynyl)-2-(4"-hexylphenylethynyl)-1,3-difluorobenzene
5-(4'-propylphenylethynyl)-2-(4"-heptylphenylethynyl)-1,3-difluorobenzene
5-(4'-propylphenylethynyl)-2-(4"-octylphenylethynyl)-1,3-difluorobenzene
5-(4'-propylphenylethynyl)-2-(4"-nonylphenylethynyl)-1,3-difluorobenzene
5-(4'-propylphenylethynyl)-2-(4"-decylphenylethynyl)-1,3-difluorobenzene
5-(4'-butylphenylethynyl)-2-(4"-methylphenylethynyl)-1,3-difluorobenzene
5-(4'-butylphenylethynyl)-2-(4"-ethylphenylethynyl)-1,3-difluorobenzene
5-(4'-butylphenylethynyl)-2-(4"-propylphenylethynyl)-1,3-difluorobenzene
C-N point 115.5°C, N-I point 180.6°C
5-(4'-butylphenylethynyl)-2-(4"-butylphenylethynyl)-1,3-difluorobenzene
C-N point 96.2°C, N-I point 167.7°C
5-(4'-butylphenylethynyl)-2-(4"-pentylphenylethynyl)-1,3-difluorobenzene
C-N point 78.3°C, N-I point 168.7°C
5-(4'-butylphenylethynyl)-2-(4"-hexylphenylethynyl)-1,3-difluorobenzene
5-(4'-butylphenylethynyl)-2-(4"-heptylphenylethynyl)-1,3-difluorobenzene
5-(4'-butylphenylethynyl)-2-(4"-octylphenylethynyl)-1,3-difluorobenzene
5-(4'-butylphenylethynyl)-2-(4"-nonylphenylethynyl)-1,3-difluorobenzene
5-(4'-butylphenylethynyl)-2-(4"-decylphenylethynyl)-1,3-difluorobenzene
5-(4'-pentylphenylethynyl)-2-(4"-methylphenylethynyl)-1,3-difluorobenzene
5-(4'-pentylphenylethynyl)-2-(4"-ethylphenylethynyl)-1,3-difluorobenzene
5-(4'-pentylphenylethynyl)-2-(4"-propylphenylethynyl)-1,3-difluorobenzene
5-(4'-pentylphenylethynyl)-2-(4"-butylphenylethynyl)-1,3-difluorobenzene
5-(4'-pentylphenylethynyl)-2-(4"-pentylphenylethynyl)-1,3-difluorobenzene
5-(4'-pentylphenylethynyl)-2-(4"-hexylphenylethynyl)-1,3-difluorobenzene
5-(4'-pentylphenylethynyl)-2-(4"-heptylphenylethynyl)-1,3-difluorobenzene
5-(4'-pentylphenylethynyl)-2-(4"-octylphenylethynyl)-1,3-difluorobenzene
5-(4'-pentylphenylethynyl)-2-(4"-nonylphenylethynyl)-1,3-difluorobenzene
5-(4'-pentylphenylethynyl)-2-(4"-decylphenylethynyl)-1,3-difluorobenzene
5-(4'-hexylphenylethynyl)-2-(4"-methylphenylethynyl)-1,3-difluorobenzene
5-(4'-hexylphenylethynyl)-2-(4"-ethylphenylethynyl)-1,3-difluorobenzene
5-(4'-hexylphenylethynyl)-2-(4"-propylphenylethynyl)-1,3-difluorobenzene
5-(4'-hexylphenylethynyl)-2-(4"-butylphenylethynyl)-1,3-difluorobenzene
5-(4'-hexylphenylethynyl)-2-(4"-pentylphenylethynyl)-1,3-difluorobenzene
5-(4'-hexylphenylethynyl)-2-(4"-hexylphenylethynyl)-1,3-difluorobenzene
5-(4'-hexylphenylethynyl)-2-(4"-heptylphenylethynyl)-1,3-difluorobenzene
5-(4'-hexylphenylethynyl)-2-(4"-octylphenylethynyl)-1,3-difluorobenzene
5-(4'-hexylphenylethynyl)-2-(4"-nonylphenylethynyl)-1,3-difluorobenzene
5-(4'-hexylphenylethynyl)-2-(4"-decylphenylethynyl)-1,3-difluorobenzene
5-(4'-heptylphenylethynyl)-2-(4"-methylphenylethynyl)-1,3-difluorobenzene
5-(4'-heptylphenylethynyl)-2-(4"-ethylphenylethynyl)-1,3-difluorobenzene

5-(4'-heptylphenylethynyl)-2-(4''-propylphenylethynyl)-1,3-difluorobenzene
5-(4'-heptylphenylethynyl)-2-(4''-butylphenylethynyl)-1,3-difluorobenzene
5-(4'-heptylphenylethynyl)-2-(4''-pentylphenylethynyl)-1,3-difluorobenzene
5-(4'-heptylphenylethynyl)-2-(4''-hexylphenylethynyl)-1,3-difluorobenzene
5-(4'-heptylphenylethynyl)-2-(4''-heptylphenylethynyl)-1,3-difluorobenzene
5-(4'-heptylphenylethynyl)-2-(4''-octylphenylethynyl)-1,3-difluorobenzene
5-(4'-heptylphenylethynyl)-2-(4''-nonylphenylethynyl)-1,3-difluorobenzene
5-(4'-heptylphenylethynyl)-2-(4''-decylphenylethynyl)-1,3-difluorobenzene
5-(4'-octylphenylethynyl)-2-(4''-methylphenylethynyl)-1,3-difluorobenzene
5-(4'-octylphenylethynyl)-2-(4''-ethylphenylethynyl)-1,3-difluorobenzene
5-(4'-octylphenylethynyl)-2-(4''-propylphenylethynyl)-1,3-difluorobenzene
5-(4'-octylphenylethynyl)-2-(4''-butylphenylethynyl)-1,3-difluorobenzene
5-(4'-octylphenylethynyl)-2-(4''-pentylphenylethynyl)-1,3-difluorobenzene
5-(4'-octylphenylethynyl)-2-(4''-hexylphenylethynyl)-1,3-difluorobenzene
5-(4'-octylphenylethynyl)-2-(4''-heptylphenylethynyl)-1,3-difluorobenzene
5-(4'-octylphenylethynyl)-2-(4''-octylphenylethynyl)-1,3-difluorobenzene
5-(4'-octylphenylethynyl)-2-(4''-nonylphenylethynyl)-1,3-difluorobenzene
5-(4'-octylphenylethynyl)-2-(4''-decylphenylethynyl)-1,3-difluorobenzene
5-(4'-nonylphenylethynyl)-2-(4''-methylphenylethynyl)-1,3-difluorobenzene
5-(4'-nonylphenylethynyl)-2-(4''-ethylphenylethynyl)-1,3-difluorobenzene
5-(4'-nonylphenylethynyl)-2-(4''-propylphenylethynyl)-1,3-difluorobenzene
5-(4'-nonylphenylethynyl)-2-(4''-butylphenylethynyl)-1,3-difluorobenzene
5-(4'-nonylphenylethynyl)-2-(4''-pentylphenylethynyl)-1,3-difluorobenzene
5-(4'-nonylphenylethynyl)-2-(4''-hexylphenylethynyl)-1,3-difluorobenzene
5-(4'-nonylphenylethynyl)-2-(4''-heptylphenylethynyl)-1,3-difluorobenzene
5-(4'-nonylphenylethynyl)-2-(4''-octylphenylethynyl)-1,3-difluorobenzene
5-(4'-nonylphenylethynyl)-2-(4''-nonylphenylethynyl)-1,3-difluorobenzene
5-(4'-nonylphenylethynyl)-2-(4''-decylphenylethynyl)-1,3-difluorobenzene
5-(4'-decylphenylethynyl)-2-(4''-methylphenylethynyl)-1,3-difluorobenzene
5-(4'-decylphenylethynyl)-2-(4''-ethylphenylethynyl)-1,3-difluorobenzene
5-(4'-decylphenylethynyl)-2-(4''-propylphenylethynyl)-1,3-difluorobenzene
5-(4'-decylphenylethynyl)-2-(4''-butylphenylethynyl)-1,3-difluorobenzene
5-(4'-decylphenylethynyl)-2-(4''-pentylphenylethynyl)-1,3-difluorobenzene
5-(4'-decylphenylethynyl)-2-(4''-hexylphenylethynyl)-1,3-difluorobenzene
5-(4'-decylphenylethynyl)-2-(4''-heptylphenylethynyl)-1,3-difluorobenzene
5-(4'-decylphenylethynyl)-2-(4''-octylphenylathynyl)-1,3-difluorobenzene
5-(4'-decylphenylethynyl)-2-(4''-nonylphenylethynyl)-1,3-difluorobenzene
5-(4'-decylphenylethynyl)-2-(4''-decylphenylethynyl)-1,3-difluorobenzene

Example 5: [Synthesis of Compound (1-e)]

Preparation of 5-(4'-propylphenylethynyl)-2-(4''-cyanophenylethynyl)-1,3-difluorobenzene

Steps 1 and 2: 4-Propylphenylacetylene was synthesized in the same manner as Steps 1 and 2 in Example 1.

Steps 3 and 4: 2-Bromo-1,3-difluoro-4-iodobenzene was synthesized in the same manner as Steps 3 and 4 in Example 4.

Step 5: 2-Bromo-1,3-difluoro-4-iodobenzene (20 g) was dissolved in diethylamine (30 ml) under nitrogen atmosphere, and then bis(triphenylphosphine)palladium(II) chloride (0.06 g) and copper(I) iodide (0.06 g) were added thereto, followed by stirring. The flask was cooled to 5°C or below, and then 4-propylphenylacetylene (10 g) was added dropwise thereto. After stirring at room temperature for 5 hours, the reaction solution was poured into a mixture of concentrated hydrochloric acid (15 ml) and ice (30 g). After extraction with chloroform, the resultant was washed with water twice, followed by distilling off chloroform. The residue was distilled under reduced pressure (b.p. 185 to 192°C/4 mmHg), and then recrystallized from a mixed solvent of acetone and methanol to give 4-bromo-3,5-difluoro-4'-propyltolane (12 g).

Steps 6 and 7: 4-Cyanophenylacetylene was synthesized in the same manner as Steps 6 and 7 in Example 2.

Step 8: 4-Bromo-3,5-difluoro-4'-propyltolane (5.0 g), 4-cyanophenylacetylene (1.9 g), triphenylphosphine (0.06 g) and bis(triphenylphosphine)palladium(II) chloride (0.04 g) were dissolved in triethylamine (33 ml) under nitrogen atmosphere, and then copper(I) iodide (0.01 g) was added thereto, followed by reflux for 5 hours. The reaction solution was poured into water (300 ml), and then extracted with chloroform. After washing the extract solution with water twice, chloroform was distilled off therefrom. The residue was purified by use of silica gel-chloroform column chromatography,

and then recrystallized from a mixed solvent of acetone and chloroform to give 5-(4'-propylphenylethynyl)-2-(4"-cyanophenylethynyl)-1,3-difluorobenzene (0.7 g). The C-N point and the N-I point of the compound were 147.5°C and 263.9°C, respectively.

The following compounds were synthesized in the same manner as the above method:
5-(4'-methylphenylethynyl)-2-(4"-cyanophenylethynyl)-1,3-difluorobenzene
5-(4'-ethylphenylethynyl)-2-(4"-cyanophenylethynyl)-1,3-difluorobenzene
5-(4'-butylphenylethynyl)-2-(4"-cyanophenylethynyl)-1,3-difluorobenzene
5-(4'-pentylphenylethynyl)-2-(4"-cyanophenylethynyl)-1,3-difluorobenzene
5-(4'-hexylphenylethynyl)-2-(4"-cyanophenylethynyl)-1,3-difluorobenzene
5-(4'-heptylphenylethynyl)-2-(4"-cyanophenylethynyl)-1,3-difluorobenzene
5-(4'-octylphenylethynyl)-2-(4"-cyanophenylethynyl)-1,3-difluorobenzene
5-(4'-nonylphenylethynyl)-2-(4"-cyanophenylethynyl)-1,3-difluorobenzene
5-(4'-decylphenylethynyl)-2-(4"-cyanophenylethynyl)-1,3-difluorobenzene

Example 6: [Synthesis of Compound (1-f)]

Preparation of 1,4-bis(4'-propylphenylethynyl)-2,5-difluorobenzene

Steps 1 and 2: 4-Propylphenylacetylene was synthesized in the same manner as Steps 1 and 2 in Example 1.

Step 3: 2,5-Difluoroaniline (38 g) was dissolved in acetic acid (120 ml), and then pyridine (25 g) was added thereto, followed by stirring. A mixed solution of iodine monochloride (50 g) with acetic acid (30 ml) was added dropwise thereto. After stirring at room temperature for 1 hour, the mixed solution was further stirred at 70 to 80°C for 2 hours. Then, the reaction solution was poured into water, and the precipitated crystals were filtered, followed by washing with water. The resulting crystals were dissolved in chloroform, and then washed with water twice, further with 10 % potassium hydroxide aqueous solution twice, and furthermore with water twice. Then the chloroform was distilled off therefrom. The residue was distilled under reduced pressure (b.p. 130 to 140°C/20 mmHg), and then recrystallized from methanol, to give 4-iodo-2,5-difluoroaniline (50 g).

Step 4: Sodium nitrite (19 g) was dissolved in sulfuric acid (148 ml), and then acetic acid (170 ml) was added thereto at 10°C or below. The mixed solution was kept at 20 to 25°C, and 4-iodo-2,5-difluoroaniline (50 g) was added thereto during 1 hour, followed by stirring for 2 hours. The reaction solution was added dropwise to a mixed solution of copper bromide (49 g) with 48 % hydrobromic acid (142 ml), and stirred overnight. Then, water (300 ml) was added to the solution, and the resulting solution was extracted with chloroform, followed by washing with water 3 times. After distilling off chloroform, the resultant was recrystallized from methanol to give 2-bromo-1,4-difluoro-4-iodobenzene (45 g).

Step 5: 2-Bromo-1,4-difluoro-4-iodobenzene (19 g) was dissolved in diethylamine (28 ml) under nitrogen atmosphere, and then bis(triphenylphosphine)palladium(II) chloride (0.06 g) and copper(I) iodide (0.06 g) were added thereto, followed by stirring. The flask was cooled to 5°C or below, and then 4-propylphenylacetylene (10 g) was added dropwise thereto. After stirring at room temperature for 5 hours, the reaction solution was poured into a mixture of concentrated hydrochloric acid (15 ml) and ice (30 g). After extraction with chloroform, the resultant was washed with water twice, followed by distilling off chloroform. The residue was distilled under reduced pressure (b.p. 170 to 180°C/4 mmHg) to give 4-bromo-2,5-difluoro-4'-propyltolane (16 g).

Steps 6 and 7: 4-Propylphenylacetylene was synthesized in the same manner as Steps 1 and 2 in Example 1.

Step 8: 4-Bromo-2,5-difluoro-4'-propyltolane (5.0 g), 4-propylphenylacetylene (2.1 g), triphenylphosphine (0.06 g) and bis(triphenylphosphine)palladium(II) chloride (0.04 g) were dissolved in triethylamine (33 ml) under nitrogen atmosphere, and then copper(I) iodide (0.01 g) was added thereto, followed by reflux for 5 hours. The reaction solution was poured into a mixture of concentrated hydrochloric acid (16 ml) and ice (30 g), and then extracted with chloroform. After washing the resultant with water twice, chloroform was distilled off therefrom. The residue was purified by use of silica gel-chloroform column chromatography, and then recrystallized from acetone, to give 1,4-bis(4'-propylphenylethynyl)-2,5-difluorobenzene (2.6 g). The C-N point and the N-I-point of the compound were 153.4°C and 197.7°C, respectively.

The following compounds were synthesized in the same manner as the above method:
1,4-bis(4'-methylphenylethynyl)-2,5-difluorobenzene
1-(4'-methylphenylethynyl)-4-(4"-ethylphenylethynyl)-2,5-difluorobenzene
1-(4'-methylphenylethynyl)-4-(4"-propylphenylethynyl)-2,5-difluorobenzene
1-(4'-methylphenylethynyl)-4-(4"-butylphenylethynyl)-2,5-difluorobenzene
1-(4'-methylphenylethynyl)-4-(4"-pentylphenylethynyl)-2,5-difluorobenzene
1-(4'-methylphenylethynyl)-4-(4"-hexylphenylethynyl)-2,5-difluorobenzene
1-(4'-methylphenylethynyl)-4-(4"-heptylphenylethynyl)-2,5-difluorobenzene
1-(4'-methylphenylethynyl)-4-(4"-octylphenylethynyl)-2,5-difluorobenzene
1-(4'-methylphenylethynyl)-4-(4"-nonylphenylethynyl)-2,5-difluorobenzene
1-(4'-methylphenylethynyl)-4-(4"-decylphenylethynyl)-2,5-difluorobenzene

1,4-bis(4'-ethylphenylethynyl)-2,5-difluorobenzene
1-(4'-ethylphenylethynyl)-4-(4"-propylphenylethynyl)-2,5-difluorobenzene
1-(4'-ethylphenylethynyl)-4-(4"-butylphenylethynyl)-2,5-difluorobenzene
1-(4'-ethylphenylethynyl)-4-(4"-pentylphenylethynyl)-2,5-difluorobenzene
1-(4'-ethylphenylethynyl)-4-(4"-hexylphenylethynyl)-2,5-difluorobenzene
1-(4'-ethylphenylethynyl)-4-(4"-heptylphenylethynyl)-2,5-difluorobenzene
1-(4'-ethylphenylethynyl)-4-(4"-octylphenylethynyl)-2,5-difluorobenzene
1-(4'-ethylphenylethynyl)-4-(4"-nonylphenylethynyl)-2,5-difluorobenzene
1-(4'-ethylphenylethynyl)-4-(4"-decylphenylethynyl)-2,5-difluorobenzene
1-(4'-propylphenylethynyl)-4-(4"-butylphenylethynyl)-2,5-difluorobenzene
C-N point 117.7°C, N-I point 183.8°C
1-(4'-propylphenylethynyl)-4-(4"-pentylphenylethynyl)-2,5-difluorobenzene
1-(4'-propylphenylethynyl)-4-(4"-hexylphenylethynyl)-2,5-difluorobenzene
1-(4'-propylphenylethynyl)-4-(4"-heptylphenylethynyl)-2,5-difluorobenzene
1-(4'-propylphenylethynyl)-4-(4"-octylphenylethynyl)-2,5-difluorobenzene
1-(4'-propylphenylethynyl)-4-(4"-nonylphenylethynyl)-2,5-difluorobenzene
1-(4'-propylphenylethynyl)-4-(4"-decylphenylethynyl)-2,5-difluorobenzene
1,4-bis(4'-butylphenylethynyl)-2,5-difluorobenzene
C-N point 101.4°C, N-I point 169.9°C
1-(4'-butylphenylethynyl)-4-(4"-pentylphenylethynyl)-2,5-difluorobenzene
1-(4'-butylphenylethynyl)-4-(4"-hexylphenylethynyl)-2,5-difluorobenzene
1-(4'-butylphenylethynyl)-4-(4"-heptylphenylethynyl)-2,5-difluorobenzene
1-(4'-butylphenylethynyl)-4-(4"-octylphenylethynyl)-2,5-difluorobenzene
1-(4'-butylphenylethynyl)-4-(4"-nonylphenylethynyl)-2,5-difluorobenzene
1-(4'-butylphenylethynyl)-4-(4"-decylphenylethynyl)-2,5-difluorobenzene
1,4-bis(4'-pentylphenylethynyl)-2,5-difluorobenzene
1-(4'-pentylphenylethynyl)-4-(4"-hexylphenylethynyl)-2,5-difluorobenzene
1-(4'-pentylphenylethynyl)-4-(4"-heptylphenylethynyl)-2,5-difluorobenzene
1-(4'-pentylphenylethynyl)-4-(4"-octylphenylethynyl)-2,5-difluorobenzene
1-(4'-pentylphenylethynyl)-4-(4"-nonylphenylethynyl)-2,5-difluorobenzene
1-(4'-pentylphenylethynyl)-4-(4"-decylphenylethynyl)-2,5-difluorobenzene
1,4-bis(4'-hexylphenylethynyl)-2,5-difluorobenzene
1-(4'-hexylphenylethynyl)-4-(4"-heptylphenylethynyl)-2,5-difluorobenzene
1-(4'-hexylphenylethynyl)-4-(4"-octylphenylethynyl)-2,5-difluorobenzene
1-(4'-hexylphenylethynyl)-4-(4"-nonylphenylethynyl)-2,5-difluorobenzene
1-(4'-hexylphenylethynyl)-4-(4"-decylphenylethynyl)-2,5-difluorobenzene
1,4-bis(4'-heptylphenylethynyl)-2,5-difluorobenzene
1-(4'-heptylphenylethynyl)-4-(4"-octylphenylethynyl)-2,5-difluorobenzene
1-(4'-heptylphenylethynyl)-4-(4"-nonylphenylethynyl)-2,5-difluorobenzene
1-(4'-heptylphenylethynyl)-4-(4"-decylphenylethynyl)-2,5-difluorobenzene
1,4-bis(4'-octylphenylethynyl)-2,5-difluorobenzene
1-(4'-octylphenylethynyl)-4-(4"-nonylphenylethynyl)-2,5-difluorobenzene
1-(4'-octylphenylethynyl)-4-(4"-decylphenylethynyl)-2,5-difluorobenzene
1,4-bis(4'-nonylphenylethynyl)-2,5-difluorobenzene
1-(4'-nonylphenylethynyl)-4-(4"-decylphenylethynyl)-2,5-difluorobenzene
1,4-bis(4'-decylphenylethynyl)-2,5-difluorobenzene

Example 7: [Synthesis of Compound (1-g)]

Preparation of 5-(4'-butylphenylethynyl)-2-(4"-cyanophenylethynyl)-1,4-difluorobenzene

Steps 1 and 2: 4-Butylphenylacetylene was synthesized in the same manner as Steps 6 and 7 in Example 4.

Steps 3 and 4: 2-Bromo-1,4-difluoro-4-iodobenzene was synthesized in the same manner as Steps 3 and 4 in Example 6.

Step 5: 2-Bromo-1,4-difluoro-4-iodobenzene (11 g) was dissolved in diethylamine (16 ml) under nitrogen atmosphere, and then bis(triphenylphosphine)palladium(II) chloride (0.03 g) and copper(I) iodide (0.03 g) were added thereto, followed by stirring. The flask was cooled to 5°C or below, and then 4-butylphenylacetylene (6.2 g) was added dropwise thereto. After stirring at room temperature for 5 hours, the reaction solution was poured into a mixture of concentrated hydrochloric acid (9 ml) and ice (20 g). After extraction with chloroform, the resultant was washed with water twice,

followed by distilling off chloroform. The residue was distilled under reduced pressure (b.p. 170 to 180°C/5 mmHg) to give 4-bromo-2,5-difluoro-4'-butyltolane (8 g).

Steps 6 and 7: 4-Cyanophenylacetylene was synthesized in the same manner as Steps 6 and 7 in Example 2.

Step 8: 4-Bromo-2,5-difluoro-4'-butyltolane (5.2 g), 4-cyanophenylacetylene (1.9 g), triphenylphosphine (0.06 g) and bis(triphenylphosphine)palladium(II) chloride (0.04 g) were dissolved in triethylamine (33 ml) under nitrogen atmosphere, and then copper(I) iodide (0.01 g) was added thereto, followed by reflux for 5 hours. The reaction solution was poured into water (300 ml), and then extracted with chloroform. After washing the reaction solution with water twice, chloroform was distilled off therefrom. The residue was purified by use of silica gel-chloroform column chromatography, and then recrystallized from a mixed solvent of acetone and chloroform to give 5-(4'-butylphenylethynyl)-2-(4"-cyanophenylethynyl)-1,4-difluorobenzene (2.9 g). The C-N point and the N-I point of the compound were 145.5°C and 250.1°C, respectively.

The following compounds were synthesized in the same manner as the above method:

5-(4'-methylphenylethynyl)-2-(4"-cyanophenylethynyl)-1,4-difluorobenzene
5-(4'-ethylphenylethynyl)-2-(4"-cyanophenylethynyl)-1,4-difluorobenzene
5-(4'-propylphenylethynyl)-2-(4"-cyanophenylethynyl)-1,4-difluorobenzene
5-(4'-pentylphenylethynyl)-2-(4"-cyanophenylethynyl)-1,4-difluorobenzene
5-(4'-hexylphenylethynyl)-2-(4"-cyanophenylethynyl)-1,4-difluorobenzene
5-(4'-heptylphenylethynyl)-2-(4"-cyanophenylethynyl)-1,4-difluorobenzene
5-(4'-octylphenylethynyl)-2-(4"-cyanophenylethynyl)-1,4-difluorobenzene
5-(4'-nonylphenylethynyl)-2-(4"-cyanophenylethynyl)-1,4-difluorobenzene
5-(4'-decylphenylethynyl)-2-(4"-cyanophenylethynyl)-1,4-difluorobenzene

Example 8: [Liquid Crystal Composition]

In order to compare the properties between the compounds of the present invention and the conventional compounds, a liquid crystal composition a which contains 10 % of a compound of Example 1 as a compound of the present invention, and comparative liquid crystal compositions b to d which contain 10 % of liquid crystal compounds (i), (ii) and (v) shown in Table 1 above, respectively, were prepared in blending ratios shown in Table 2 below, by use of cyclohexanecarboxylic acid phenyl ester derivates, so-called ECH series liquid crystals as base liquid crystals.

Table 2

| Liquid Crystal Composition | a | b | c | d |
|---|---|---|---|---|
| 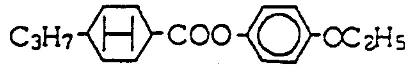 | 6.6 | 6.6 | 6.6 | 6.6 |
| 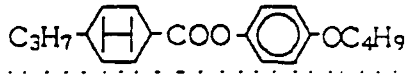 | 17.4 | 17.4 | 17.4 | 17.4 |
| 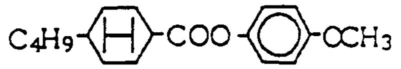 | 13.5 | 13.5 | 13.5 | 13.5 |
| 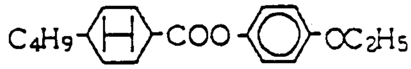 | 13.6 | 13.6 | 13.6 | 13.6 |
| 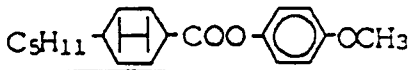 | 14.9 | 14.9 | 14.9 | 14.9 |
| 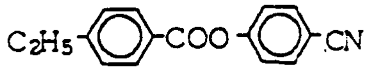 | 12.0 | 12.0 | 12.0 | 12.0 |
| 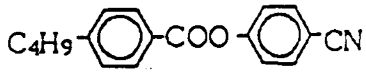 | 12.0 | 12.0 | 12.0 | 12.0 |
| 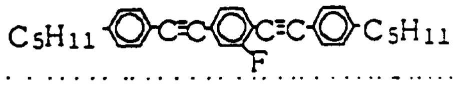 | 10.0 | | | |
| 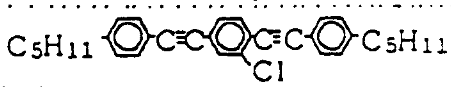 | | 10.0 | | |
| 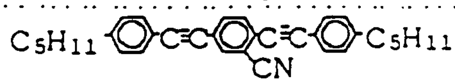 | | | 10.0 | |
| 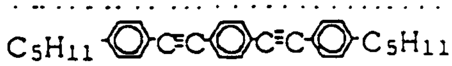 | | | | 10.0 |
| 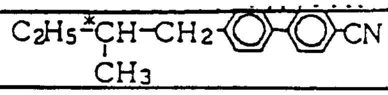 | 0.08 | 0.08 | 0.08 | 0.08 |

The composition ratio is indicated by wt%.

In addition, for further comparison, liquid crystal compositions e to l which contain 10 to 30 % of compounds of Examples 1 to 7 as compounds of the present invention, and comparative liquid crystal compositions m to o which contain 10 to 30 % of conventional liquid crystal compounds currently used for increasing the the Δn in general, were prepared in blending ratios shown in Table 3 below, by use of ECH series liquid crystals as base liquid crystals. In Table 3, the composition ratio of each compounds is indicated by wt%.

## Table 3

| Liquid Crystal Composition | e | f | g | h |
|---|---|---|---|---|
| 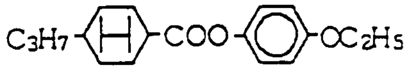 | 5.5 | 5.7 | 5.7 | 4.8 |
| 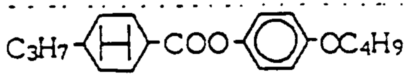 | 14.2 | 14.7 | 14.7 | 12.6 |
| 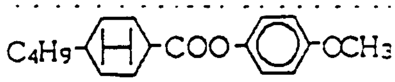 | 11.1 | 11.5 | 11.5 | 9.9 |
| 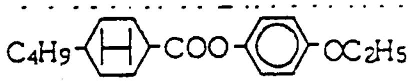 | 11.1 | 11.5 | 11.5 | 9.9 |
| 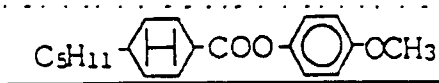 | 12.1 | 12.6 | 12.6 | 10.8 |
| 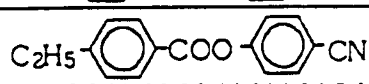 | 12.0 | 12.0 | | 10.0 |
| 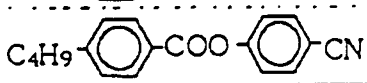 | 12.0 | 12.0 | | 10.0 |
| 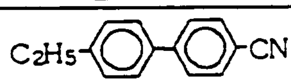 | 6.0 | | 12.0 | 6.0 |
| 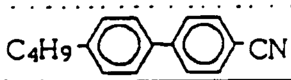 | 6.0 | | 12.0 | 6.0 |
| 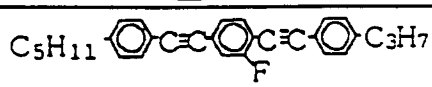 | 5.0 | 10.0 | 10.0 | 10.0 |
| 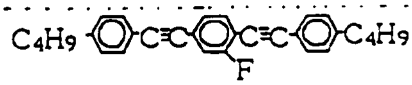 | | 10.0 | | 5.0 |
| 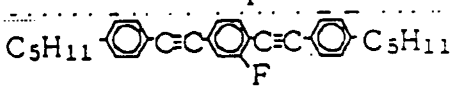 | | | 10.0 | 5.0 |
| 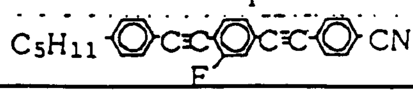 | 5.0 | | | |
| 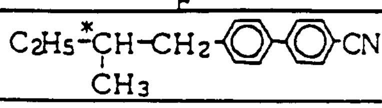 | 0.08 | 0.08 | 0.08 | 0.08 |

Table 3 (continued)

| Liquid Crystal Composition | i | j | k | l |
|---|---|---|---|---|
| 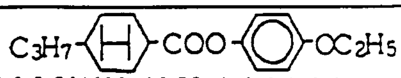 | 5.2 | 5.2 | 4.7 | 4.2 |
| 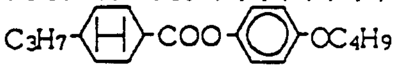 | 13.7 | 13.7 | 12.4 | 11.0 |
| 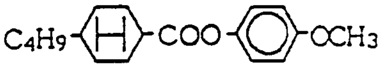 | 10.7 | 10.7 | 9.6 | 8.6 |
| 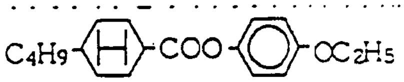 | 10.7 | 10.7 | 9.7 | 8.7 |
| 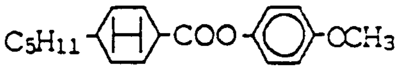 | 11.7 | 11.7 | 10.6 | 9.5 |
| 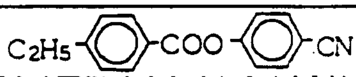 | 8.0 | 8.0 | 8.0 | 8.0 |
| 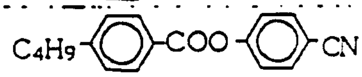 | 8.0 | 8.0 | 8.0 | 8.0 |
| 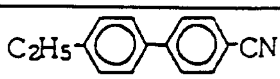 | 6.0 | 6.0 | 6.0 | 6.0 |
| 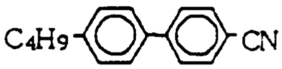 | 6.0 | 6.0 | 6.0 | 6.0 |
| 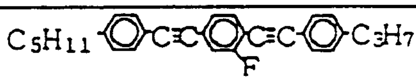 | | | 10.0 | 10.0 |
| 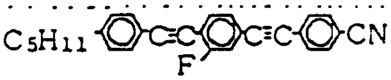 | 5.0 | | 5.0 | 5.0 |
| 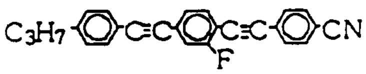 | 5.0 | | | |
| 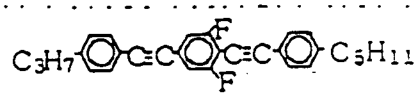 | 5.0 | 5.0 | 5.0 | 5.0 |
| 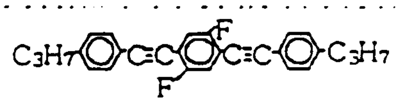 | 5.0 | 5.0 | 5.0 | 5.0 |
| 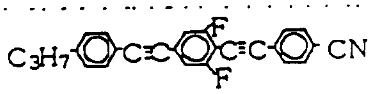 | | 5.0 | | 5.0 |
| 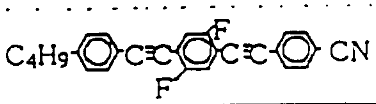 | | 5.0 | | |
| 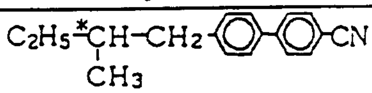 | 0.08 | 0.08 | 0.08 | 0.08 |

Table 3 (continued)

| Liquid Crystal Composition | m | n | o |
|---|---|---|---|
| 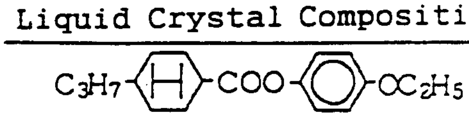 | 5.5 | 5.5 | 4.6 |
| 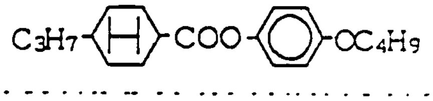 | 14.5 | 14.5 | 11.8 |
| 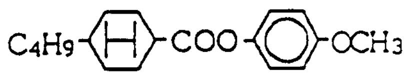 | 11.3 | 11.3 | 9.2 |
| 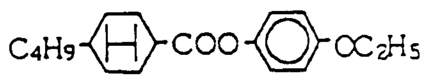 | 11.3 | 11.3 | 9.3 |
| 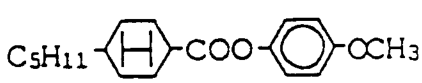 | 12.4 | 12.4 | 10.1 |
| 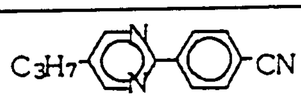 | 5.0 | 5.0 | 5.0 |
| 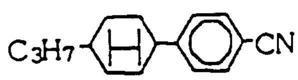 | 5.0 | 10.0 | 10.0 |
| 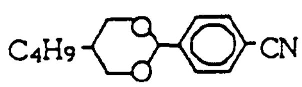 | 15.0 | 10.0 | 10.0 |
| 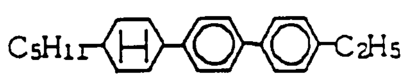 | 10.0 | | |
| 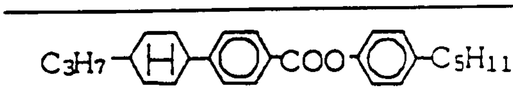 | | 10.0 | 15.0 |
| 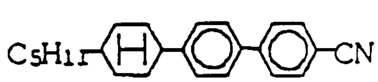 | 10.0 | | 15.0 |
| 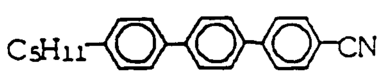 | | 10.0 | |
| 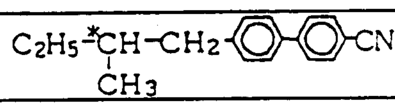 | 0.08 | 0.08 | 0.08 |

The N-I points and the Δn) of the compositions a to o were measured. The results are shown in Table 4 below.

Table 4

| Liquid Crystal Composition | a | b | c | d | e |
|---|---|---|---|---|---|
| N-I point (°C) | 70.3 | 67.8 | 65.4 | 72.6 | 65.1 |
| $\Delta n$ | 0.139 | 0.135 | 0.134 | n.d. | 0.156 |
| Liquid Crystal Composition | f | g | h | i | j |
| N-I point (°C) | 81.6 | 79.4 | 77.2 | 80.5 | 78.2 |
| $\Delta n$ | 0.177 | 0.187 | 0.185 | 0.195 | 0.192 |
| Liquid Crystal Composition | k | l | m | n | o |
| N-I point (°C) | 85.6 | 93.1 | 79.2 | 80.1 | 92.6 |
| $\Delta n$ | 0.209 | 0.232 | 0.119 | 0.127 | 0.128 |

Now, the liquid crystal composition a is compared with the comparative liquid crystal compositions b to d. The N-I point of the composition a is higher than those of the compositions b and c. On the other hand, the composition d precipitated crystals at room temperature, and consequently $\Delta n$ was impossible to be measured. Accordingly, it is proved that the liquid crystal composition a which contains a compound of the present invention exhibits a wider liquid crystal temperature range than the comparative liquid crystal compositions b to d which contain conventional compounds shown in Table 1.

Next, the liquid crystal compositions e to l which contain the compounds of the present invention are compared with the comparative liquid crystal compositions m to o which contain the conventional compounds. As the result, it is proved that the the values of $\Delta n$ of the liquid crystal compositions e to l are larger than those of the comparative liquid crystal compositions m to o by 0.03 to 0.11.

Example 9: [Liquid Crystal Display Device]

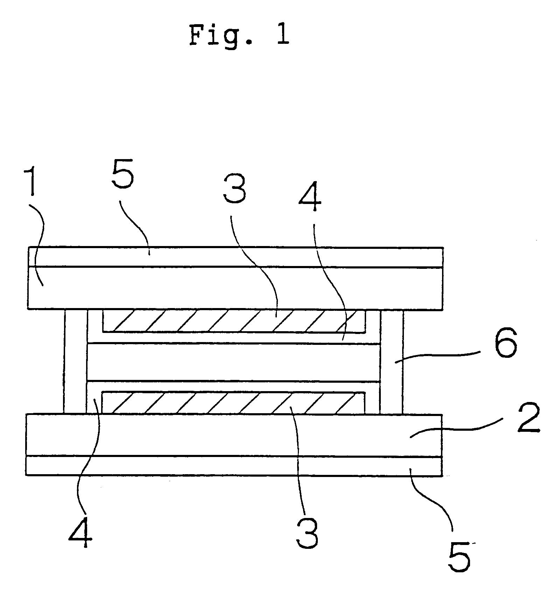

Fig. 1 is a diagrammatic sectional view of liquid crystal cell. As shown in Fig. 1, electrodes 3 consisting of transparent conductive films (e.g., ITO films) were formed on each of two glass substrates 1 and 2, and then coated with alignment layers 4 consisting of, for example, polyimide. Next, the alignment layers 4 were subjected to an orientation treatment by rubbing. Subsequently, the glass substrates 1 and 2 were placed to have their electrode carrying sides facing each other and were bonded together through a sealing material 6. A plurality of sample cells were prepared, and then the liquid crystal compositions a to o prepared in Example 8 were injected into the gap between the glass substrates of a respective sample cell. This was followed by pasting a polarization (?) plate 5 outside of substrate 1 and a reflection-type polarization plate 5 outside of substrate 2, thereby to obtain TN mode-liquid crystal display panels A to O. The cell gaps d of the liquid crystal display panels A to C were set to 9.0μm, and the cell gaps d of the liquid crystal display panels E to O were set so that in each case the value of $\Delta n \cdot d$ was in the range of about 1.25 to 1.35 μm. For each liquid crystal display cell thus obtained we measured the threshold voltage (hereinafter referred to as Vth), the visual angle dependency of the voltage-light transmittance (hereinafter referred to as $\alpha$), the sharpness (hereinafter referred to as $\beta$), the rise time (hereinafter referred to as Tr) and the drop time (i.e., the fall time; hereinafter referred to as Td). These measurements were performed on the reflection-type sample cells by use of an alternating current static drive. The values of $\alpha$, $\beta$ and Vth were determined according to the following equations:

$$\alpha = \frac{V10\ (\theta=80°,\ T=25°C)}{V10\ (\theta=50°,\ T=25°C)}$$

$$\beta = \frac{V10\ (\theta=80°,\ T=25°C)}{V90\ (\theta=80°,\ T=25°C)}$$

$$Vth = V10$$

with $\theta$: the angle of incident light against cell (the direction vertical to the panel is defined as $\theta$ = 90°); V10, V90: the voltage values that resulted in 10 % and 90 % of light transmittance, respectively.

The rise time Tr is the time that elapses after a voltage change from V90 to V10 until the transmittance is reduced to 10 % (θ = 80°), and the fall time Td is the time required to recover the transmittance to 90 % when the voltage is changed from V10 to V90 (θ = 80°).

The results are shown in Table 5 below.

Table 5

| Liquid Crystal Panel | A | B | C | D | E |
|---|---|---|---|---|---|
| Liquid Crystal Composition | a | b | c | d | e |
| Cell Gap (μm) | 9.0 | 9.0 | 9.0 | - | 8.0 |
| Δn·d | 1.26 | 1.22 | 1.21 | - | 1.25 |
| Vth (V) | 1.621 | 1.690 | 1.583 | - | 1.347 |
| α | 1.170 | 1.164 | 1.161 | - | 1.190 |
| β | 1.247 | 1.264 | 1.266 | - | 1.280 |
| Tr (ms) | 134 | 129 | 144 | - | 100 |
| Td (ms) | 212 | 183 | 210 | - | 132 |
| Tr + Td (ms) | 346 | 312 | 354 | - | 232 |

Table 5 (Continued)

| Liquid Crystal Panel | F | G | H | I | J |
|---|---|---|---|---|---|
| Liquid Crystal Composition | f | g | h | i | j |
| Cell Gap (μm) | 7.0 | 7.0 | 7.0 | 7.0 | 7.0 |
| Δn·d | 1.24 | 1.31 | 1.30 | 1.37 | 1.34 |
| Vth (V) | 1.633 | 1.995 | 1.519 | 1.516 | 1.488 |
| α | 1.163 | 1.149 | 1.180 | 1.186 | 1.189 |
| β | 1.264 | 1.247 | 1.270 | 1.278 | 1.278 |
| Tr (ms) | 79 | 65 | 71 | 73 | 71 |
| Td (ms) | 109 | 92 | 96 | 90 | 107 |
| Tr + Td (ms) | 188 | 157 | 167 | 163 | 178 |

Table 5 (Continued)

| Liquid Crystal Panel | K | L | M | N | O |
|---|---|---|---|---|---|
| Liquid Crystal Composition | k | l | m | n | o |
| Cell Gap (μm) | 6.0 | 6.0 | 10.0 | 7.0 | 7.0 |
| Δn·d | 1.25 | 1.39 | 1.19 | 1.37 | 1.34 |
| Vth (V) | 1.637 | 1.611 | 1.645 | 1.516 | 1.488 |
| α | 1.189 | 1.194 | 1.170 | 1.186 | 1.189 |
| β | 1.270 | 1.280 | 1.259 | 1.278 | 1.278 |
| Tr (ms) | 54 | 55 | 128 | 139 | 141 |
| Td (ms) | 81 | 90 | 189 | 191 | 196 |
| Tr + Td (ms) | 135 | 145 | 317 | 330 | 337 |

By comparison of the liquid crystal panel A which used a liquid crystal composition containing a liquid crystal compound of the present invention with the comparative liquid crystal panels B and D which used liquid crystal compositions containing the conventional liquid crystal compounds shown in Table 1, it is proved that the liquid crystal panel A has a smaller β value than the comparative liquid crystal panels B and D. Accordingly, the liquid crystal panel A can be driven by high time-sharing addressing in the TN mode display.

Furthermore, a comparison of the liquid crystal panels E to L which used liquid crystal compositions containing liquid crystal compounds of the present invention with the comparative liquid crystal panels M to O which used liquid crystal compositions containing the conventional liquid crystal compounds, proves that the liquid crystal panels E to L have smaller Tr + Td values than the comparative liquid crystal panels M to O by 50 to 150 ms.

When a STN mode liquid crystal display cell was used instead of the TN mode liquid crystal display cell used in above Examples, the results obtained were similar to the above results.

As mentioned above, the compounds of the present invention have an excellent compatibility with other liquid crystal compounds, and consequently are very effective for providing liquid crystal compositions of a wide practical temperature range and a large value of Δn by blending them with the general liquid crystal mixtures.

Accordingly, by using liquid crystal compositions containing a liquid crystal compound of the present invention, a liquid crystal display device which exhibits a wide practical temperature range and a high-speed response can be prepared.

The compounds according to the present invention are extremely useful as basic components of liquid crystal compositions which are used in STN mode display systems which are currently the most widely used systems.

## Claims

1. A tolane derivative represented by the following general formula:

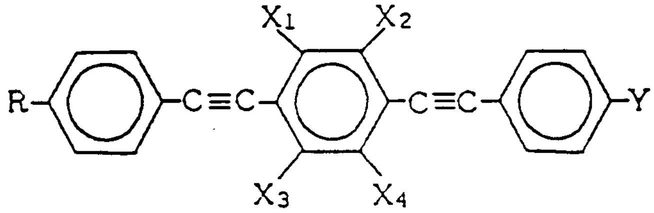

(1)

wherein R represents a straight-chain alkyl group having 1 to 10 carbon atoms, each of $X_1$, $X_2$, $X_3$ and $X_4$ represents a fluorine atom or a hydrogen atom wherein at least one of $X_1$ to $X_4$ represents a fluorine atom, and Y represents a nitrile group or a straight-chain alkyl group having 1 to 10 carbon atoms.

**2.** The tolane derivative according to Claim 1 represented by any one of the following general formulae:

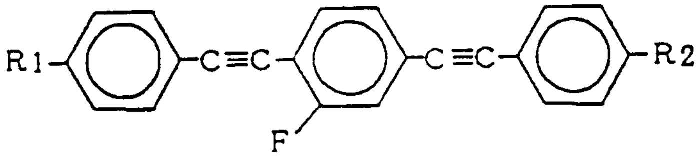

(1-a)

wherein each of R1 and R2 represents a straight-chain alkyl group having 1 to 10 carbon atoms,

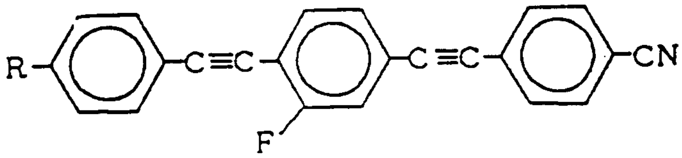

(1-b)

wherein R represents a straight-chain alkyl group having 1 to 10 carbon atoms,

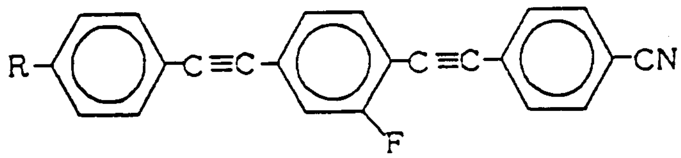

(1-c)

wherein R represents a straight-chain alkyl group having 1 to 10 carbon atoms,

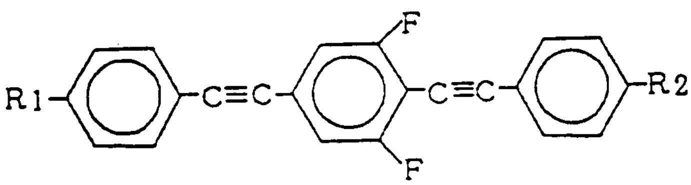

(1-d)

wherein each of R1 and R2 represents a straight-chain alkyl group having 1 to 10 carbon atoms,

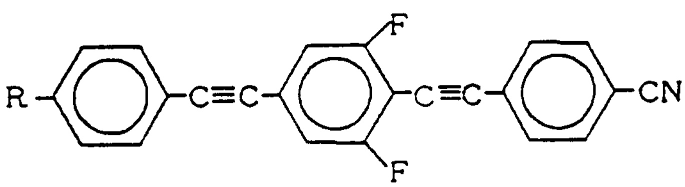

(1-e)

wherein R represents a straight-chain alkyl group having 1 to 10 carbon atoms,

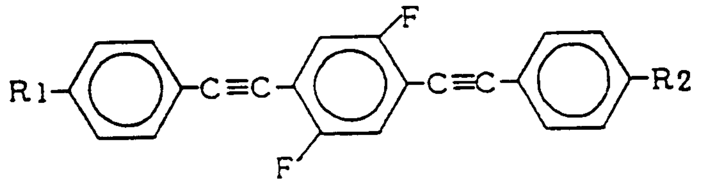

(1-f)

wherein each of R1 and R2 represents a straight-chain alkyl group having 1 to 10 carbon atoms, and

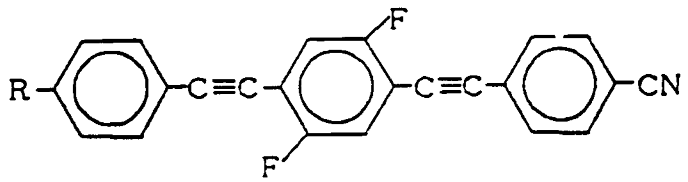

(1-g)

wherein R represents a straight-chain alkyl group having 1 to 10 carbon atoms.

3. A liquid crystal composition containing at least one tolane derivative as defined in claim 1 or 2.

4. The liquid crystal composition according to Claim 3, which contains:
5 to 50 wt% of at least one tolane derivative as defined in claim 1 or 2; and
30 to 80 wt% of at least one cyclohexanecarboxylic acid phenyl ester derivative represented by the following general formula:

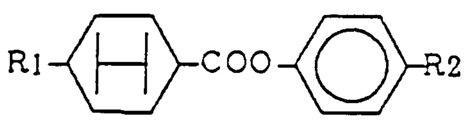

wherein each of R1 and R2 represents a straight-chain alkyl group having 1 to 10 carbon atoms, and the cyclohexane ring is in trans configuration.

5. The liquid crystal composition according to Claim 3, which contains:
10 to 35 wt% of at least one tolane derivative as defined in claim 1 or 2;
40 to 70 wt% of at least one cyclohexanecarboxylic acid phenyl ester derivative represented by the following general formula:

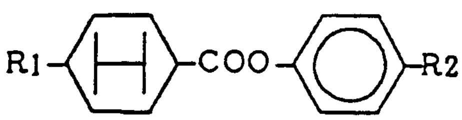

wherein each of R1 and R2 represents a straight-chain alkyl group having 1 to 10 carbon atoms and the cyclohexane ring is in trans configuration; and
10 to 50 wt% of at least one compound exhibiting a positive dielectric anisotropy represented by the following general

formulae:

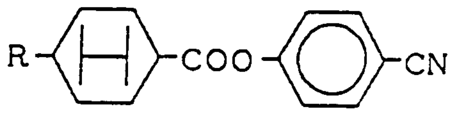

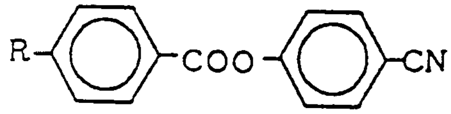

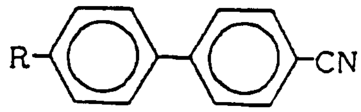

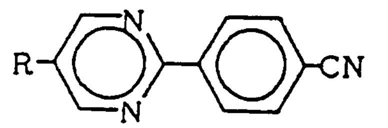

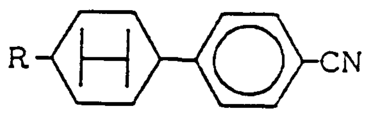

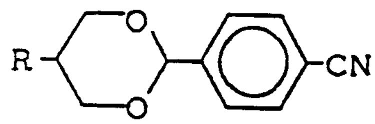

wherein R represents a straight-chain alkyl group having 1 to 10 carbon atoms and the cyclohexane ring is in trans configuration.

6. A liquid crystal display device using a liquid crystal composition as defined in any one of claims 3 to 5.

**Patentansprüche**

1. Tolanderivat der folgenden allgemeinen Formel:

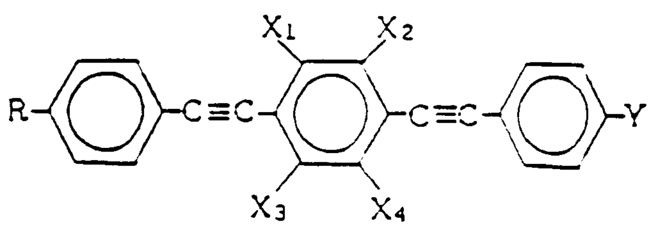

(1)

worin R eine geradkettige Alkylgruppe mit 1 bis 10 Kohlenstoffatomen bedeutet, $X_1$, $X_2$, $X_3$ und $X_4$ jeweils ein Fluoratom oder ein Wasserstoffatom bedeuten, wobei mindestens einer der Reste $X_1$ bis $X_4$ ein Fluoratom bedeutet, und Y eine Nitrilgruppe oder eine geradkettige Alkylgruppe mit 1 bis 10 Kohlenstoffatomen bedeutet.

2. Tolanderivat nach Anspruch 1 der folgenden allgemeinen Formeln:

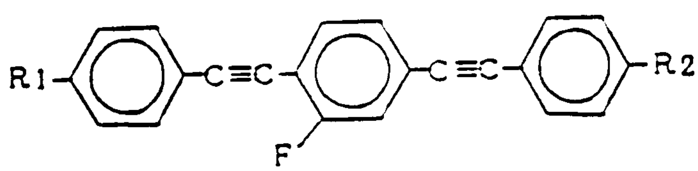

(1-a)

worin R1 und R2 eine geradkettige Alkylgruppe mit 1 bis 10 Kohlenstoffatomen bedeuten,

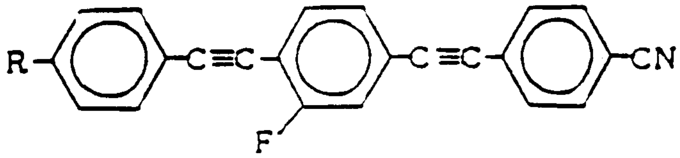

(1-b)

worin R eine geradkettige Alkylgruppe mit 1 bis 10 Kohlenstoffatomen bedeutet,

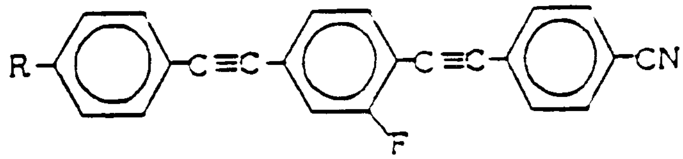

(1-c)

worin R eine geradkettige Alkylgruppe mit 1 bis 10 Kohlenstoffatomen bedeutet,

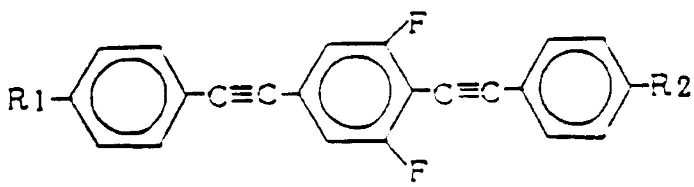

(1-d)

worin R1 und R2 jeweils eine geradkettige Alkylgruppe mit 1 bis 10 Kohlenstoffatomen bedeuten,

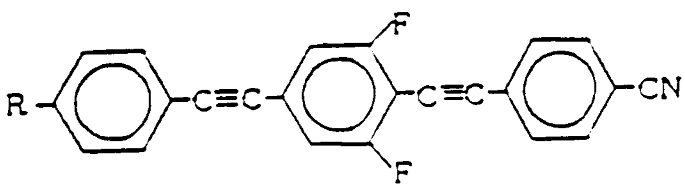

(1-e)

worin R eine geradkettige Alkylgruppe mit 1 bis 10 Kohlenstoffatomen bedeutet,

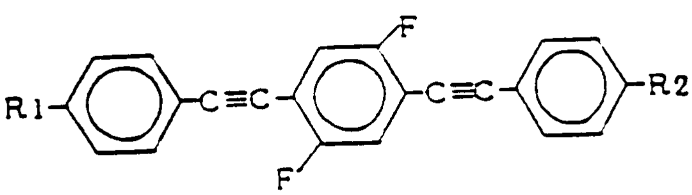

(1-f)

worin R1 und R2 eine geradkettige Alkylgruppe mit 1 bis 10 Kohlenstoffatomen bedeuten und

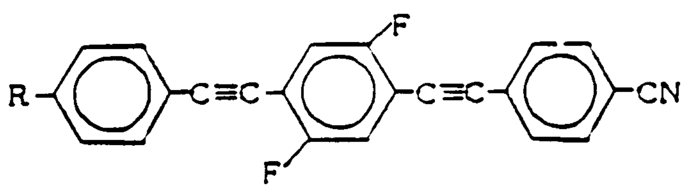

(1-g)

worin R eine geradkettige Alkylgruppe mit 1 bis 10 Kohlenstoffatomen bedeutet.

**3.** Flüssigkristall-Zusammensetzung, enthaltend mindestens ein Tolanderivat nach Anspruch 1 oder 2.

**4.** Flüssigkristall-Zusammensetzung nach Anspruch 3, enthaltend:

5 bis 50 Gew.-% mindestens eines Tolanderivats nach Anspruch 1 oder 2; und

30 bis 80 Gew.-% mindestens eines Cyclohexancarbonsäurephenylesterderivats der folgenden allgemeinen Formel:

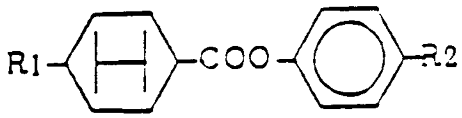

worin R1 und R2 jeweils eine geradkettige Alkylgruppe mit 1 bis 10 Kohlenstoffatomen bedeuten und der Cyclohexanring sich in trans-Konfiguration befindet.

**5.** Flüssigkristall-Zusammensetzung nach Anspruch 3, enthaltend:

10 bis 35 Gew.-% mindestens eines Tolanderivats nach Anspruch 1 oder 2;

40 bis 70 Gew.-% mindestens eines Cyclohexancarbonsäurephenylesterderivats der folgenden allgemeinen Formel:

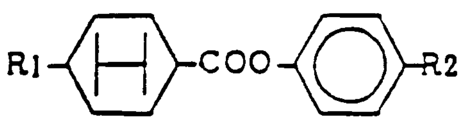

worin R1 und R2 jeweils eine geradkettige Alkylgruppe mit 1 bis 10 Kohlenstoffatomen bedeuten und der Cyclohexanring sich in trans-Konfiguration befindet; und

10 bis 50 Gew.-% mindestens einer Verbindung mit einer positiven dielektrischen Anisotropie der folgenden allgemeinen Formeln:

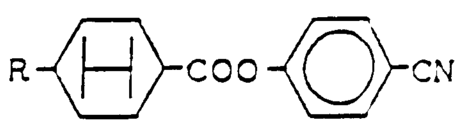

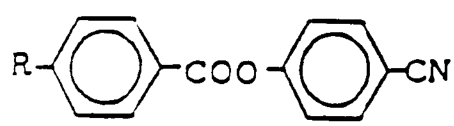

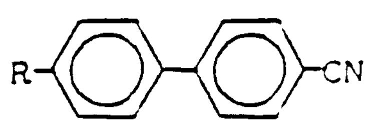

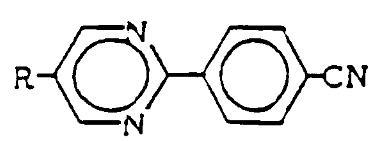

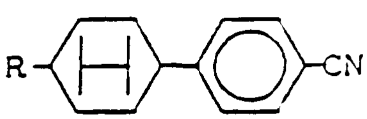

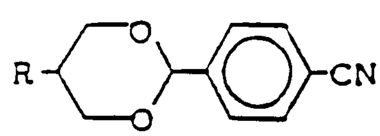

worin R eine geradkettige Alkylgruppe mit 1 bis 10 Kohlenstoffatomen bedeutet und der Cyclohexanring sich in trans-Konfiguration befindet.

**6.** Flüssigkristall-Anzeigevorrichtung unter Verwendung einer Flüssigkristall-Zusammensetzung nach einem der Ansprüche 3 bis 5.

## Revendications

1. Dérivé du tolane répondant à la formule générale suivante :

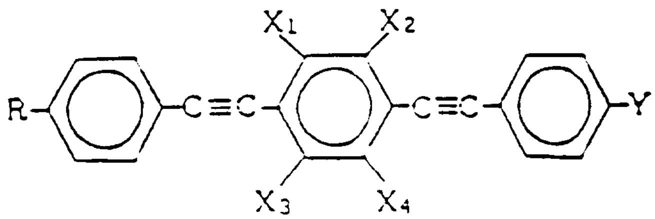

(1)

dans laquelle R représente un groupe alkyle à chaîne linéaire en $C_1$-$C_{10}$, chacun des symboles $X_1$, $X_2$, $X_3$ et $X_4$ représente un atome de fluor ou un atome d'hydrogène, au moins un des symboles $X_1$ à $X_4$ représentant un atome de fluor, et Y représente un groupe nitrile ou un groupe alkyle à chaîne linéaire en $C_1$-$C_{10}$.

2. Dérivé du tolane selon la revendication 1, répondant à l'une des formules générales suivantes :

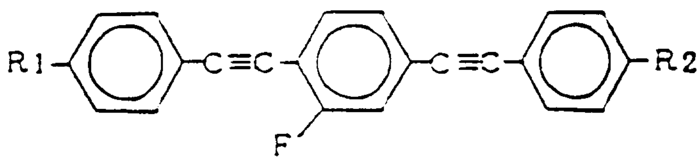

(1-a)

dans laquelle chacun des symboles R1 et R2 représente un groupe alkyle à chaîne linéaire en $C_1$-$C_{10}$,

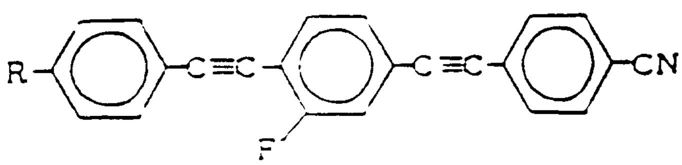

(1-b)

dans laquelle R représente un groupe alkyle à chaîne linéaire en $C_1$-$C_{10}$,

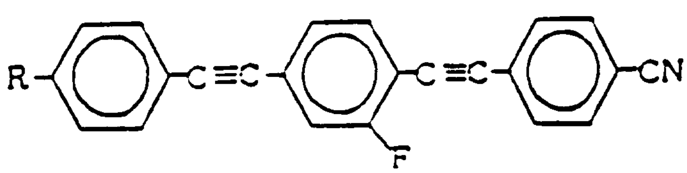

(1-c)

dans laquelle R représente un groupe alkyle à chaîne linéaire en $C_1$-$C_{10}$,

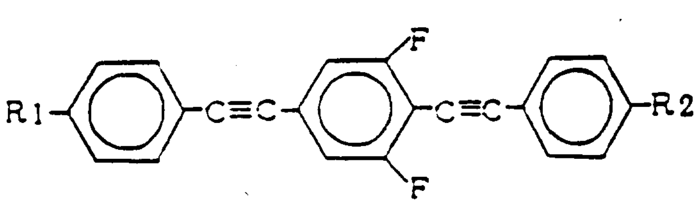

(1-d)

dans laquelle chacun des symboles R1 et R2 représente un groupe alkyle à chaîne linéaire en $C_1$-$C_{10}$,

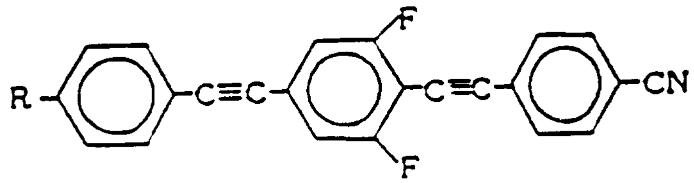

(1-e)

dans laquelle R représente un groupe alkyle à chaîne linéaire en $C_1$-$C_{10}$,

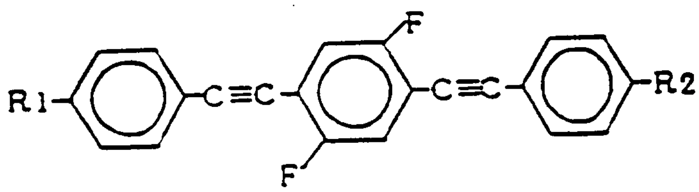

(1-f)

dans laquelle chacun des symboles R1 et R2 représente un groupe alkyle à chaîne linéaire en $C_1$-$C_{10}$, et

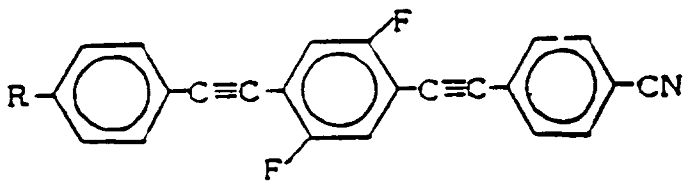

(1-g)

dans laquelle R représente un groupe alkyle à chaîne linéaire en $C_1$-$C_{10}$.

3. Composition de cristaux liquides contenant au moins un dérivé du tolane selon les revendications 1 ou 2.

4. Composition de cristaux liquides selon la revendication 3, qui contient :
5 à 50 % en poids d'au moins un dérivé du tolane tel que défini dans les revendications 1 ou 2 ; et
30 à 80 % en poids d'au moins un ester phénylique d'acide cyclohexane carboxylique répondant à la formule générale suivante :

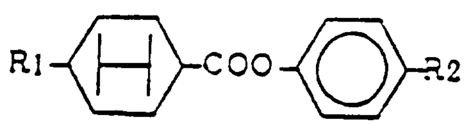

dans laquelle chacun des symboles R1 et R2 représente un groupe alkyle à chaîne linéaire en $C_1$-$C_{10}$, et le cycle cyclohexane est dans la configuration trans.

5. Composition de cristaux liquides selon la revendication 3, qui contient :
10 à 30 % en poids d'au moins un dérivé du tolane tel que défini dans les revendications 1 ou 2 ;
40 à 70 % en poids d'au moins un ester phénylique d'acide cyclohexane carboxylique répondant à la formule générale suivante :

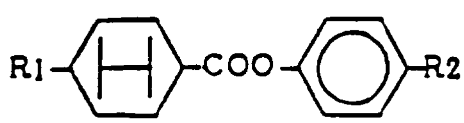

dans laquelle chacun des symboles R1 et R2 représente un groupe alkyle à chaîne linéaire en $C_1$-$C_{10}$, et le cycle cyclohexane est dans la configuration trans ; et

10 à 50 % en poids d'au moins un composé présentant une anisotropie diélectrique positive répondant aux formules générales suivantes :

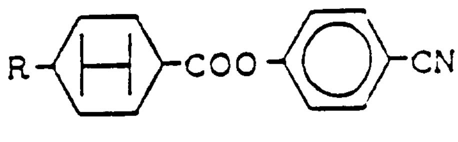

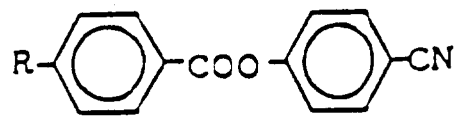

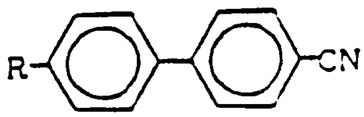

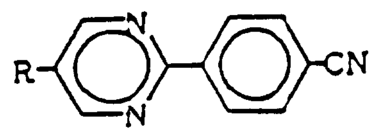

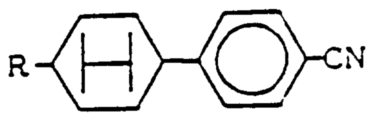

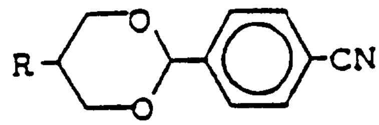

dans lesquelles R représente un groupe alkyle à chaîne linéaire en $C_1$-$C_{10}$, et le cycle cyclohexane est dans la configuration trans.

**6.** Dispositif d'affichage à cristaux liquides utilisant une composition de cristaux liquides telle que définie dans l'une quelconque des revendications 3 à 5.

Fig. 1